# EUROPEAN PATENT APPLICATION

(11) **EP 4 628 489 A1**
(43) Date of publication of application: **08.10.2025**
(21) Application number: 23896808.5
(22) Date of filing: 29.11.2023
(51) Int. Cl.: C07D 471/04, A61K 31/437, A61K 31/438, A61K 31/444, A61K 31/506, A61P 21/00, A61P 35/00

(54) **7-AZAINDOLE COMPOUNDS, PREPARATION METHOD THEREFOR, AND USE THEREOF**

(30) Priority: 01.12.2022 CN 202211536668; 27.11.2023 CN 202311597376
(71) Applicant: Fudan University, Shanghai 200433 (CN); East China Normal University, Shanghai 200062 (CN)
(72) Inventor: DONG, Xiaochun, Shanghai 200433 (CN); ZHANG, Xiongwen, Shanghai 200062 (CN); ZHAO, Weili, Shanghai 200433 (CN); SUN, Weikuan, Shanghai 200062 (CN); RUAN, Li, Shanghai 200433 (CN); WANG, Xiaoting, Shanghai 200062 (CN); FAN, Rong, Shanghai 200433 (CN); GU, Xiaofan, Shanghai 200062 (CN)
(74) Representative: Callaghan, Dayle Anne
(86) International application number: PCT/CN2023/134906
(87) International publication number: WO 2024/114661

(57) **Abstract**

Disclosed in the present invention are 7-azaindole compounds, a preparation method therefor, and the use thereof, belonging to the field of drug synthesis. The present invention particularly relates to 7-azaindole compounds containing pyrazole or indole substitution represented by general formula (I), a preparation method therefor and the use thereof in medicine. The compounds of the present invention alleviate cancer cachexia skeletal muscle atrophy by means of inhibiting ActRIIB protein in the MSTN pathway, thereby achieving a remarkable anti-cancer-cachexia effect. Experimental results show that the compounds have good anti-cachexia activity and thus can be further used for preparing novel anti-cachexia drugs.

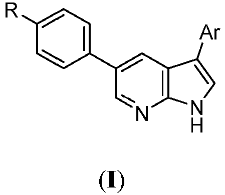

## Description

### TECHNICAL FIELD

The present invention belongs to the field of drug synthesis and relates to a novel 7-azaindole compound, a preparation method therefor and the use thereof. Specifically, it relates to 7-azaindole compounds containing pyrazole or indole substitution, and a preparation method therefor and the use thereof in medicine.

### BACKGROUND ART

Cachexia is a progressive wasting syndrome accompanied by systemic functional failure such as emaciation, anemia, and mental depression. It is mostly caused by cancer and other serious chronic diseases (such as chronic obstructive pulmonary disease, chronic heart failure, AIDS). Among others, cachexia caused by cancer is called cancer cachexia. Cachexia affects nearly 80% of patients with advanced cancer and causes approximately 30% of cancer deaths. It not only seriously affects the quality of life of cancer patients, but also weakens the effects of chemotherapy and radiotherapy, greatly reducing the survival time of patients. Cancer cachexia has gradually attracted widespread attention. However, the pathogenesis of cancer cachexia is extremely complex, which has led to the fact that most of the recommended treatments for cachexia today are limited to increasing appetite, nutritional intervention, etc. Currently, the only drug approved for the treatment of cancer cachexia is anamorelin (ghrelin receptor agonist), and the options are very limited.

Inhibiting muscle atrophy is currently considered to be one of the important methods for treating and alleviating cancer cachexia. In recent years, studies have found that myostatin (MSTN)-related pathways play an important role in cancer cachexia, and high expression of myostatin is positively correlated with skeletal muscle atrophy in patients with cancer cachexia. Myostatin, also known as growth differentiation factor 8 (GDF-8), is a protein mainly secreted by skeletal muscle and plays a negative regulatory role in muscle growth and development. MSTN is also expressed in adipose tissue and plays an important regulatory role in fat deposition. The C-terminus of the MSTN homodimer first binds to an activin type II receptor (ActRII, mainly ActRIIB) on the cell membrane surface, and then recruits an ActR type I receptor to form a receptor complex, which in turn phosphorylates the transcription factor Smad2/3 in the cytoplasm. The activated Smad complex enters the nucleus and activates various forms of protein degradation. Inhibiting any part of the MSTN-ActRIIB-Smad signaling pathway can effectively affect the biological function of MSTN. In addition, after binding to the ActR IIB receptor, MSTN can also regulate the expression of its downstream target genes through various non-Smad protein signal transduction pathways such as MAPK, Erkl/2, and JNK, thereby producing biological functions.

Currently, the drugs under development targeting MSTN-related pathways mainly include the following types of drugs: ① antibody inhibitors of myostatin: Landogrozumab (LY2495655), Domagrozumab (PF-06252616) and Stamulumab (MYO-029); ② Myostatin's propeptides: AMG-745; ③ ActRIIB-Fc recombinant fusion proteins: Ramatercept (ACE-031); ④ antibody inhibitors of ActRIIB receptors: Bimagrumab (BYM338); ⑤ gene therapy with follistatin (natural inhibitor of MSTN) as the core. Some peptide inhibitors of MSTN have also been reported in the literature. The antibody inhibitor CDD866 of ActRIIB can effectively block the Myostatin-ActRIIB signaling pathway. In animal experiments, it can effectively combat the cancer cachexia produced by the CT26 mouse colon cancer model. In combination with chemotherapy drugs, it can alleviate the muscle wasting caused by chemotherapy and significantly prolong survival. Bimagrumab, an antibody inhibitor of ActRIIB under development by Novartis, was found in multiple Phase II clinical trials to be able to effectively increase muscle volume (5.0-7.8%) and exercise capacity in cachectic patients at a dose of 30 mg/kg.

The above research results confirm that inhibiting the ActRIIB receptor has a significant clinical effect in delaying cancer cachexia, but the relevant drugs under development are currently all protein or gene drugs. The research difficulty and overall cost of such large molecule drugs are relatively high, which brings difficulties for their application in clinical treatment. Therefore, the research on small molecule inhibitors of ActRIIB protein will surely bring new hope and opportunities to the research on anti-cancer-cachexia drugs.

### SUMMARY OF THE INVENTION

An objective of the present invention is to provide a novel 7-azaindole compound with good ActRIIB inhibitory effect, and specifically the present invention relates to a 7-azaindole compound containing a pyrazole or indole substituent.

Another objective of the present invention is to provide a method for preparing the above-mentioned 7-azaindole compound, and in particular to a method for preparing a 7-azaindole compound containing a pyrazole ring.

The 7-azaindole compound of the present invention has the following general formula (I): 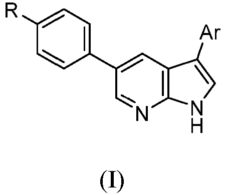 wherein:
Ar is pyrazole, indole or pyrazole containing substituents; in the pyrazole containing substituents, the substituent is selected from C1-4 hydrocarbon groups, and the number of substituents is 1 or 2;
R is 4-methylpiperazine, piperazine, morpholine, 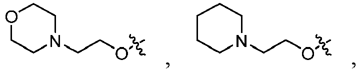 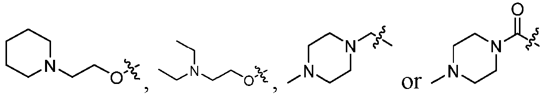
optionally, with the restrictions that:
   a) when R is morpholine, Ar is not 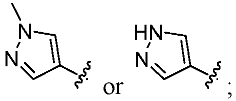
   b) when R is 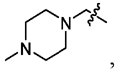 Ar is not 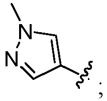
   c) when Ar is indole, R is not

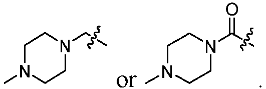

In some embodiments of the present invention, the structural formula of the 7-azaindole compound is as shown in formula (II): 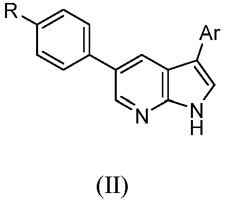 or a pharmaceutically acceptable salt thereof, wherein:
Ar is selected from pyrazole, indole, pyrazole containing substituents, isoxazole containing substituents, pyrimidine or 3-pyridyl; in the pyrazole containing substituents and the isoxazole containing substituents, the substituent is selected from C1-4 hydrocarbon groups, and the number of substituents is 1 or 2;
R is selected from 4-methylpiperazine, piperazine, morpholine, 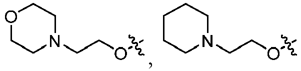 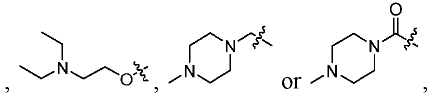
optionally, with the restrictions that:
   a) when R is morpholine, Ar is not 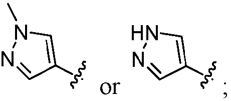
   b) when R is 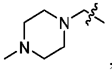 Ar is not 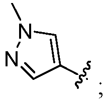
   c) when Ar is indole, R is not

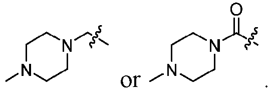

In some embodiments of the present invention, the 7-azaindole compound is a compound as represented by structural formula (III): 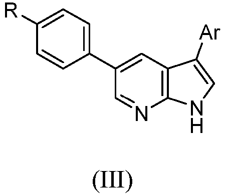 or a pharmaceutically acceptable salt thereof, wherein:
Ar is selected from pyrazole, indole, pyrazole containing substituents, isoxazole containing substituents, pyrimidine or 3-pyridine; in the pyrazole containing substituents and the isoxazole containing substituents, the substituent is selected from C1-4 hydrocarbon groups, and the number of substituents is 1 or 2;
R is selected from 4-methylpiperazine, piperazine,

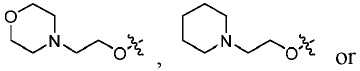

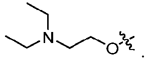

The present invention further relates to a pharmaceutical composition comprising structural formula (IV): 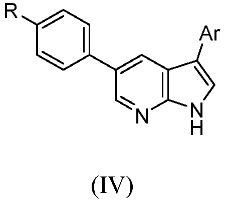 or a pharmaceutically acceptable salt thereof, and a pharmaceutically acceptable additive, preferably the additive being selected from a pharmaceutically acceptable carrier, diluent or excipient; wherein:
Ar is selected from pyrazole, indole, pyrazole containing substituents, isoxazole containing substituents, pyrimidine or 3-pyridine; in the pyrazole containing substituents and the isoxazole containing substituents, the substituent is selected from C1-4 hydrocarbon groups, and the number of substituents is 1 or 2;
R is selected from 4-methylpiperazine, piperazine, morpholine, 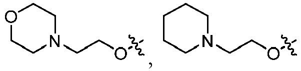 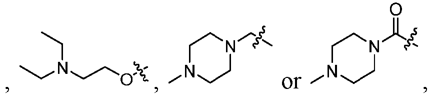
optionally, with the restrictions that:
   a) when R is morpholine, Ar is not 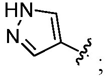
   b) when R is 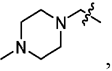 Ar is not 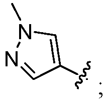
   c) when Ar is indole, R is not 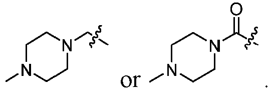 The present invention further relates to the use of a compound represented by structural formula (V): 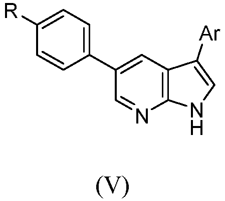
or a pharmaceutically acceptable salt thereof in the preparation of anti-cachexia drugs, wherein:
   Ar is selected from pyrazole, indole, pyrazole containing substituents, isoxazole containing substituents, pyrimidine, pyridine or phenyl; in the pyrazole containing substituents and the isoxazole containing substituents, the substituent is selected from C1-4 hydrocarbon groups, and the number of substituents is 1 or 2;
   R is selected from 4-methylpiperazine, piperazine, morpholine, 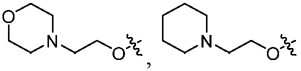 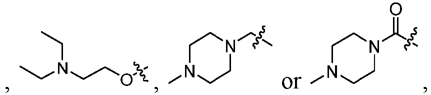
   optionally, with the restrictions that:
      a) when R is morpholine, Ar is not 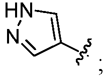
      b) when R is 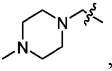 Ar is not 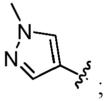
      c) when Ar is indole, R is not

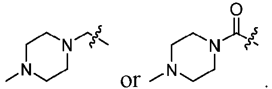

In some preferred embodiments of the present invention, in formulas (I), (II), (III), (IV), (V) or pharmaceutically acceptable salts thereof,
the Ar is indole. Further preferably, the indole is

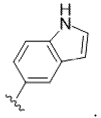

In some preferred embodiments of the present invention, in formulas (I), (II), (III), (IV), (V) or pharmaceutically acceptable salts thereof,
the Ar is pyrazole or pyrazole containing substituents.

In some preferred embodiments of the present invention, in formulas (I), (II), (III), (IV), (V) or pharmaceutically acceptable salts thereof
the Ar is selected from 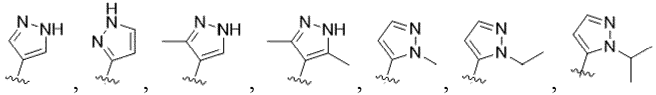 or

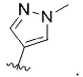

Further preferably, the Ar is

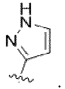

In some preferred embodiments of the present invention, in formulas (I), (II), (III), (IV), (V) or pharmaceutically acceptable salts thereof
the R is 4-methylpiperazine. Further preferably, the R is

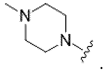

The 7-azaindole compounds of the present invention are selected from the following compounds: 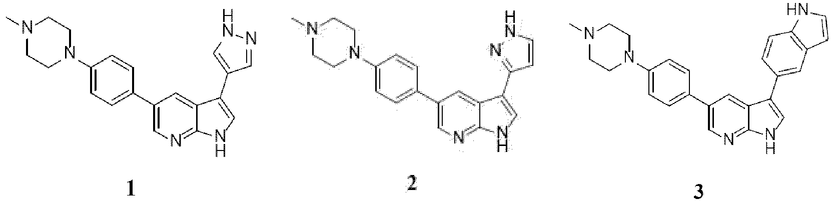 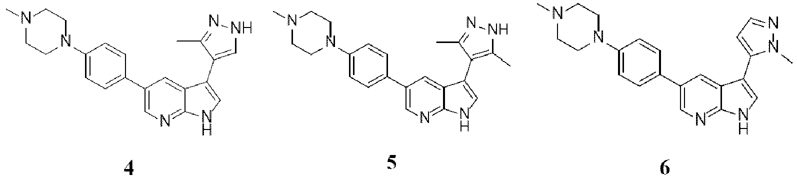 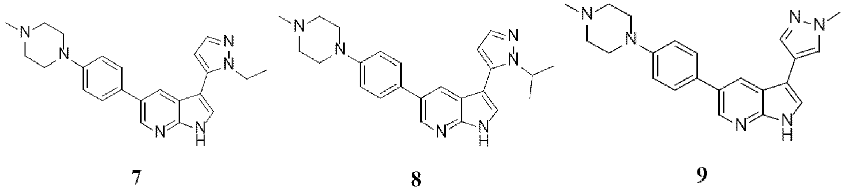 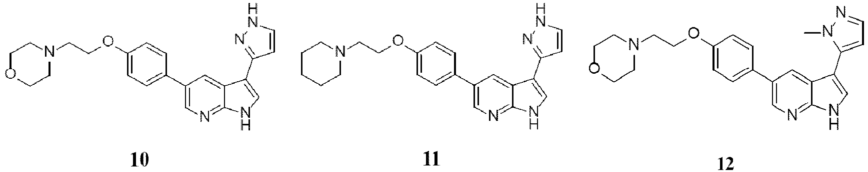 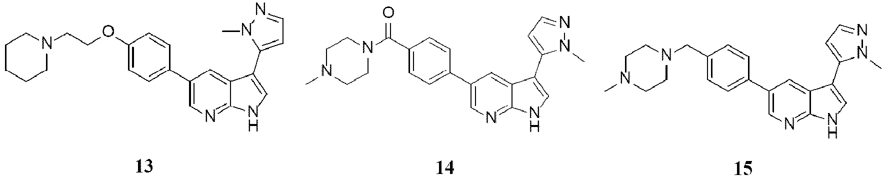 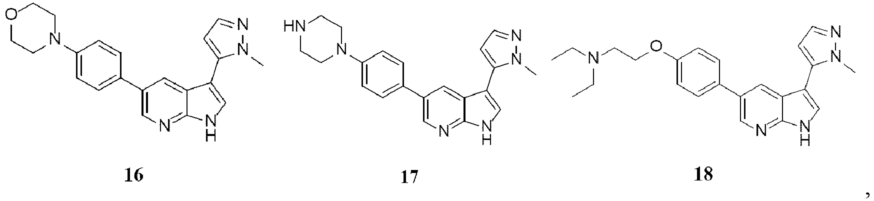 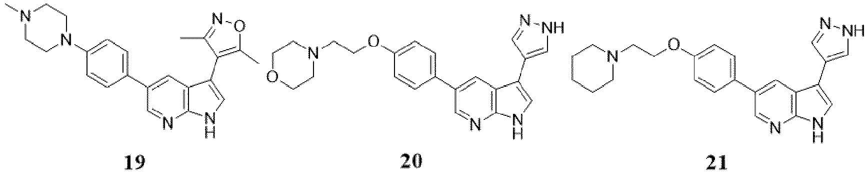 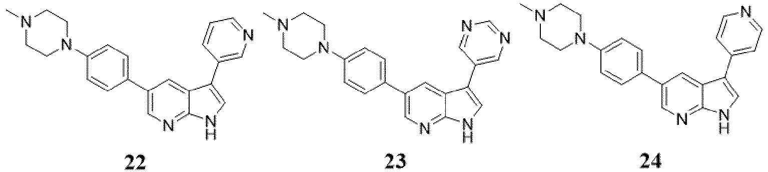 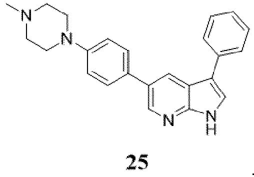 or a pharmaceutically acceptable salt thereof.

The present invention further relates to 7-azaindole compounds of formulas (I), (II), (III), (IV), (V) or any one of compounds 1-25 or pharmaceutically acceptable salts thereof for use in inhibiting ActRIIB activity in a subject in need thereof.

The present invention further relates to 7-azaindole compounds of formulas (I), (II), (III), (IV), (V) or any one of compounds 1-25 or pharmaceutically acceptable salts thereof for use in treating cachexia. Preferably it is used to treat cancer cachexia.

The present invention further relates to a pharmaceutical composition, characterized in that it comprises (I), (II), (III), (IV), (V) or any one of compounds 1-25 or pharmaceutically acceptable salts thereof, and a pharmaceutically acceptable additive.

In some embodiments of the present invention, the pharmaceutically acceptable additive is selected from pharmaceutically acceptable carriers, diluents or excipients.

The present invention further relates to the use of 7-azaindole compounds of formulas (I), (II), (III), (IV), (V) or any one of compounds 1-25 or pharmaceutically acceptable salts thereof or the composition of the present invention as described above in the preparation of ActRIIB inhibitors.

The present invention further relates to the use of 7-azaindole compounds of formulas (I), (II), (III), (IV), (V) or any one of compounds 1-25 or pharmaceutically acceptable salts thereof in the preparation of anti-cachexia drugs, especially anti-cancer-cachexia drugs.

Taking compound 2 as an example, the preparation process of compounds 1-11 of the present invention is as follows:

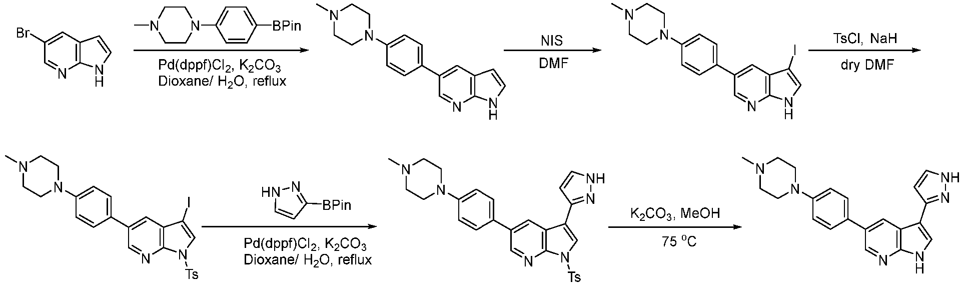

Taking compound 12 as an example, the preparation process of compounds 12-17 of the present invention is as follows:

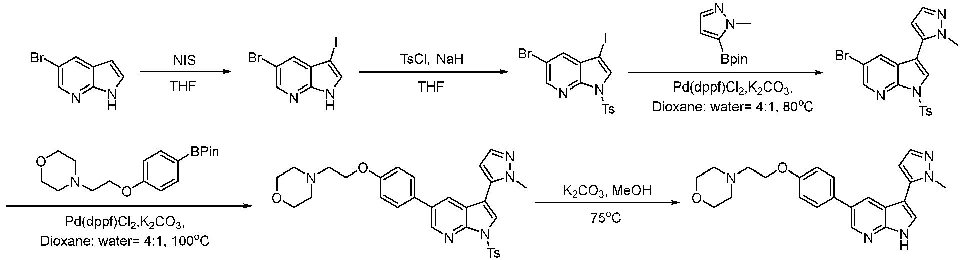

The preparation process of compound 18 of the present invention is as follows:

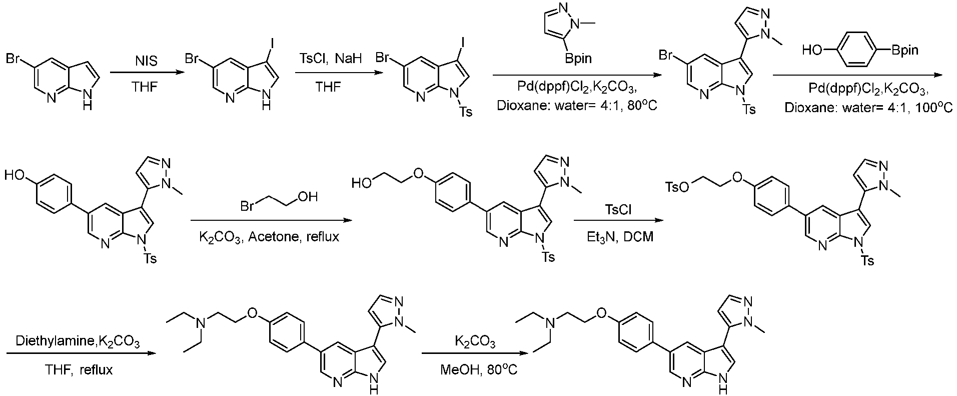

The present invention further provides the use of 7-azaindole compounds or pharmaceutically acceptable salts thereof in the preparation of anti-cachexia drugs.

The present invention provides a composition and use of the composition in the preparation of anti-cachexia drugs. The composition comprises the 7-azaindole compound or a pharmaceutically acceptable salt thereof, and a pharmaceutically acceptable additive, wherein the additive is selected from pharmaceutically acceptable carriers, diluents or excipients.

Furthermore, the anti-cachexia drug is selected from anti-cancer-cachexia drugs.

The compounds of the present invention were subjected to in vivo and in vitro experiments and found to be able to alleviate muscle atrophy caused by cancer cachexia, and animal experiments showed that they could significantly alleviate weight loss caused by cancer cachexia, indicating that such 7-azaindole compounds have anti-cancer-cachexia effects, can be used to treat cancer cachexia and related diseases, and are ideal drugs for treating cancer cachexia.

### BRIEF DESCRIPTION OF THE DRAWINGS

FIG. 1 shows the HE staining image of the control group in Example 26.
FIG. 2 shows the HE staining image of the model group in Example 26.
FIG. 3 shows the HE staining image of experimental group 1 in Example 26.
FIG. 4 shows the HE staining image of experimental group 2 in Example 26.
FIG. 5 shows the HE staining image of experimental group 3 in Example 26.
FIG. 6 shows the HE staining image of experimental group 4 in Example 26.
FIG. 7 shows the HE staining image of experimental group 5 in Example 26.
FIG. 8 shows the HE staining image of experimental group 6 in Example 26.
FIG. 9 shows the HE staining image of experimental group 7 in Example 26.
FIG. 10 shows the HE staining image of experimental group 8 in Example 26.
FIG. 11 shows the HE staining image of experimental group 9 in Example 26.
FIG. 12 shows the HE staining image of experimental group 10 in Example 26.
FIG. 13 shows the HE staining image of experimental group 11 in Example 26.
FIG. 14 shows the HE staining image of experimental group 12 in Example 26.
FIG. 15 shows the HE staining image of experimental group 13 in Example 26.
FIG. 16 shows the HE staining image of experimental group 14 in Example 26.
FIG. 17 shows the HE staining image of experimental group 15 in Example 26.
FIG. 18 shows the HE staining image of experimental group 16 in Example 26.
FIG. 19 shows the HE staining image of experimental group 17 in Example 26.
FIG. 20 shows the HE staining image of experimental group 18 in Example 26.
FIG. 21 shows the HE staining image of experimental group 19 in Example 26.
FIG. 22 shows the HE staining image of experimental group 20 in Example 26.
FIG. 23 shows the HE staining image of experimental group 21 in Example 26.
FIG. 24 shows the HE staining image of experimental group 22 in Example 26.
FIG. 25 shows the HE staining image of experimental group 23 in Example 26.
FIG. 26 shows the HE staining image of experimental group 24 in Example 26.
FIG. 27 shows the HE staining image of experimental group 25 in Example 26.
FIG. 28 shows the HE staining image of experimental group 26 in Example 26.
FIG. 29 shows the HE staining image of experimental group 27 in Example 26.
FIG. 30 shows the HE staining image of experimental group 28 in Example 26.
FIG. 31 shows the HE staining image of experimental group 29 in Example 26.
FIG. 32 shows the HE staining image of experimental group 30 in Example 26.
FIG. 33 shows the mouse tumor-bearing body weight change curve of compound 2 in Example 28.
FIG. 34 shows the mouse tumor-free body weight change curve of compound 2 in Example 28.
FIG. 35 shows the mouse body weight change rate curve of compound 2 in Example 28.
FIG. 36 shows the mouse tumor volume change curve of compound 2 in Example 28.
FIG. 37 shows the mouse tumor photograph of compound 2 in Example 28.
FIG. 38 shows the mouse average accumulative food intake change curve of compound 2 in Example 28.
FIG. 39 shows the results of the mouse muscle grasp force test of compound 2 in Example 28.
FIG. 40 shows the results of the mouse right hind limb mass test of compound 2 in Example 28.
FIG. 41 shows the photograph of the mouse right hind limb of compound 2 in Example 28.
FIG. 42 shows the results of the mouse gastrocnemius mass test of compound 2 in Example 28.
FIG. 43 shows the photograph of the mouse gastrocnemius of compound 2 in Example 28.
FIG. 44 shows the results of the mouse tibialis anterior mass test of compound 2 in Example 28.
FIG. 45 shows the photograph of the mouse tibialis anterior of compound 2 in Example 28.
FIG. 46 shows the mouse tumor-bearing body weight change curve of compounds 3 and 10 in Example 28.
FIG. 47 shows the mouse tumor-free body weight change curve of compounds 3 and 10 in Example 28.
FIG. 48 shows the mouse body weight change rate curve of compounds 3 and 10 in Example 28.
FIG. 49 shows the mouse tumor volume change curve of compounds 3 and 10 in Example 28.
FIG. 50 shows the mouse tumor photograph of compounds 3 and 10 in Example 28.
FIG. 51 shows the mouse average accumulative food intake change curve of compounds 3 and 10 in Example 28.
FIG. 52 shows the results of the mouse muscle grasp force test of compounds 3 and 10 in Example 28.
FIG. 53 shows the results of the mouse right hind limb mass test of compounds 3 and 10 in Example 28.
FIG. 54 shows the photograph of the mouse right hind limb of compounds 3 and 10 in Example 28.
FIG. 55 shows the results of the mouse tibialis anterior mass test of compounds 3 and 10 in Example 28.
FIG. 56 shows the photograph of the mouse tibialis anterior of compounds 3 and 10 in Example 28.
FIG. 57 shows the results of the mouse gastrocnemius mass test of compounds 3 and 10 in Example 28.
FIG. 58 shows the photograph of the mouse gastrocnemius of compounds 3 and 10 in Example 28.
FIG. 59 is a schematic diagram of mouse gastrocnemius tissue sections of compounds 3 and 10 in Example 28.
FIG. 60 is a statistical diagram of the cross-sectional distribution of mouse gastrocnemius fibers of compounds 3 and 10 in Example 28.

### DETAILED DESCRIPTION

The present invention will be further described below in conjunction with specific embodiments. It should be understood that the following embodiments are only used to illustrate the present invention rather than to limit the scope of the present invention, and those skilled in the art may make some non-essential improvements and adjustments based on the above contents of invention.

In the present invention, the pharmacodynamic test methods used are methods well known to those skilled in the art.

In the present invention, the C2C12 cells, C26 cells and Balb/c mice used can be obtained by those skilled in the art through commercial routes.

### Example 1: Synthesis of compound 1, 3-(1H-pyrazol-4-yl)-5-[4-(4-methylpiperazin-1-yl)phenyl]-1H-pyrrolo[2,3-b]pyridine

### 1) Synthesis of 5-[4-(4-methylpiperazin-1-yl)phenyl]-1H-pyrrolo[2,3-b]pyridine:

Into a 50 mL two-necked flask, 5-bromo-7-azaindole (1.0 eq., 10.15 mmol, 2.00 g), 4-(4-methylpiperazin-1-yl)phenylboronic acid pinacol ester (1.5 eq., 15.23 mmol, 4.60 g), potassium carbonate (3.0 eq., 30.45 mmol, 4.21 g), [1,1'-bis(diphenylphosphino)ferrocene]dichloropalladium (II) (0.05 eq., 0.51 mmol, 368 mg) were added, and then 15 mL of 1,4-dioxane and 5 mL of water were added. The atmosphere was replaced with argon three times, and then the system was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 1.87 g of 5-[4-(4-methylpiperazin-1-yl)phenyl]-1*H*-pyrrolo[2,3-b]pyridine with a yield of 63.2%.

### 2) Synthesis of 3-iodo-5-[4-(4-methylpiperazin-1-yl)phenyl]-1H-pyrrolo[2,3-b]pyridine:

Into a 50 mL two-necked flask, 5-[4-(4-methylpiperazin-1-yl)phenyl]-1*H*-pyrrolo[2,3-b]pyridine (1.0 eq., 5.13 mmol, 1.50 g) was added, then 15 mL of anhydrous DMF was added, N-iodosuccinimide (1.1 eq., 5.64 mmol, 1.27 g) was slowly added in batches, and the system was allowed to react for 30 mins. After the reaction stopped, saturated sodium thiosulfate solution was added to quench the reaction, and then a large amount of water was added to precipitate the product. The product was directly filtered without other post-treatment to obtain 2.04 g of crude product 3-iodo-5-[4-(4-methylpiperazin-1-yl)phenyl]-1*H*-pyrrolo[2,3-b]pyridine with a yield of 95.9%.

### 3) Synthesis of 3-iodo-1-[(4-methylphenyl)dioxo-λ⁶-thio]-5-[4-(4-methylpiperazin-1-yl)phenyl]pyrrolo[2,3-b]pyridine:

Into a 50 mL two-necked flask, 3-iodo-5-[4-(4-methylpiperazin-1-yl)phenyl]-1*H*-pyrrolo[2,3-b]pyridine (1.0 eq., 4.78 mmol, 2.00 g) was added, and 15 mL of anhydrous DMF was added. In ice bath, sodium hydride (60%, dispersed in liquid paraffin) (1.1 eq., 5.26 mmol, 126 mg) was slowly added and the system was allowed to react for 30 mins. Then, p-toluenesulfonyl chloride (1.3 eq., 6.22 mmol, 1.19 g) was slowly added and the reaction was carried out at room temperature for 4 h. After the reaction was completed, ice water was added to quench the reaction. The product precipitated and was directly filtered to obtain a crude product, which was recrystallized to obtain 2 g of 3-iodo-1-[(4-methylphenyl)dioxo-λ⁶-thio]-5-[4-(4-methylpiperazin-1-yl)phenyl]pyrrolo[2, 3-*b*]pyridine with a yield of 74.6%.

### 4) Synthesis of 3-(1H-pyrazol-4-yl)-1-[(4-methylphenyl)dioxo-λ⁶-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-b]pyridine (1.0 eq., 0.26 mmol, 150 mg), 1-Boc-pyrazole-4-boronic acid pinacol ester (1.5 eq., 0.39 mmol, 116 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 87 mg of 3-(1*H*-pyrazol-4-yl)-1-[(4-methylphenyl)dioxo-λ⁶-thio]-5-[4-(4-methylpiperazin-1-yl)phenyl]pyrrolo[2,3-*b*]pyridine with a yield of 65.4%. ¹H NMR (600 MHz, Chloroform-*d*) δ 8.63 (d, *J* = 2.0 Hz, 1H), 8.09 (d, *J =* 8.1 Hz, 2H), 8.03 (d, *J* = 2.0 Hz, 1H), 7.92 (s, 2H), 7.80 (s, 1H), 7.43 (d, *J =* 8.3 Hz, 2H), 7.27 (s, 2H), 6.97 (d, *J =* 8.3 Hz, 2H), 3.36 (t, *J =* 4.9 Hz, 4H), 2.81 (t, *J =* 4.8 Hz, 4H), 2.52 (s, 3H), 2.35 (s, 3H).¹³C NMR (151 MHz, CDCl₃) δ 150.30, 146.39, 145.27, 144.06, 135.33, 132.52, 131.86, 129.74, 129.60, 128.12, 127.96, 126.15, 122.15, 121.89, 116.54, 112.95, 111.89, 54.53, 48.07, 45.38, 21.64.

### 5) Synthesis of 3-(1H-pyrazol-4-yl)-5-[4-(4-methylpiperazin-1-yl)phenyl]-1H-pyrrolo[2,3-b]pyridine

Into a 10 mL single-necked flask, 3-(1*H*-pyrazol-4-yl)-1-[(4-methylphenyl)dioxo-λ⁶-thio]-5-[4-(4-methylpiperazin-1-yl)phenyl]pyrrolo[2,3-*b*]pyridine (1.0 eq., 0.17 mmol, 87 mg) and potassium carbonate (3.0 eq., 0.51 mmol, 70 mg) were added, and 5 mL of methanol was added. The mixture was allowed to react at 80°C for 4 h. After concentration under reduced pressure, it was purified by column chromatography (DCM:MeOH=10:1) to obtain 44 mg of 3-(1*H*-pyrazol-4-yl)-5-[4-(4-methylpiperazin-1-yl)phenyl]-1*H*-pyrrolo[2,3-*b*]pyridine with a yield of 72.4%. ¹H NMR (600 MHz, DMSO-d₆) δ 8.48 (s, 1H), 8.29 (s, 1H), 8.09 (s, 2H), 7.70 (s, 1H), 7.66 (d, *J =* 8.2 Hz, 2H), 7.06 (d, *J =* 8.2 Hz, 2H), 3.25 (s, 4H), 2.66 (s, 4H), 2.36 (s, 3H). ¹³C NMR (151 MHz, DMSO) δ 145.08, 143.23, 136.70, 125.04, 123.62, 123.41, 122.98, 120.87, 119.82, 117.88, 112.89, 111.25, 109.77, 102.25, 49.43, 42.90, 40.38.

### Example 2: Synthesis of compound 2, 3-(1H-pyrazol-3-yl)-5-[4-(4-methylpiperazin-1-yl)phenyl]-1H-pyrrolo[2,3-b]pyridine

### 1) Synthesis of 3-(1H-pyrazol-3-yl)-1-[(4-methylphenyl)dioxo-λ⁶-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶ -thio]-5-[4-(4-methylpiperazin-1-yl)phenyl]pyrrolo[2,3-*b*]pyridine (1.0 eq., 0.26 mmol, 150 mg), 1*H*-pyrazole-3-boronic acid pinacol ester (1.5 eq., 0.39 mmol, 76 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 83 mg of 3-(1*H*-pyrazol-3-yl)-1-[(4-methylphenyl)dioxo-λ⁶-thio]-5-[4-(4-methylpiperazin-1-yl)phenyl]pyrrolo[2,3-*b*]pyridine with a yield of 62.4%. ¹H NMR (600 MHz, Chloroform-*d*) δ 8.66 (d, *J* = 2.5 Hz, 1H), 8.59 (s, 1H), 8.09 (d, *J =* 8.0 Hz, 2H), 8.01 (d, *J* = 2.0 Hz, 1H), 7.65 (d, *J* = 2.6 Hz, 1H), 7.52 - 7.44 (m, 2H), 7.25 (d, *J =* 6.7 Hz, 2H), 7.00 - 6.94 (m, 2H), 6.62 (d, *J =* 2.5 Hz, 1H), 3.29 (t, *J =* 5.0 Hz, 4H), 2.64 (t, *J =* 5.0 Hz, 4H), 2.39 (s, 3H), 2.34 (s, 3H).¹³C NMR (151 MHz, CDCl₃) δ 150.61, 146.49, 145.21, 144.16, 135.36, 132.74, 130.30, 129.69, 129.38, 128.23, 128.07, 128.01, 123.30, 121.29, 116.23, 113.27, 103.04, 54.90, 48.58, 45.94, 21.63.

### 2) Synthesis of 3-(1H-pyrazol-3-yl)-5-[4-(4-methylpiperazin-1-yl)phenyl]-1H-pyrrolo[2,3-b]pyridine

Into a 10 mL single-necked flask, 3-(1*H*-pyrazol-3-yl)-1-[(4-methylphenyl)dioxo-λ⁶-thio]-5-[4-(4-methylpiperazin-1-yl)phenyl]pyrrolo[2,3-*b*]pyridine (1.0 eq., 0.16 mmol, 83 mg) and potassium carbonate (3.0 eq., 0.48mmol, 67mg) were added, and 5 mL of methanol was added. The mixture was allowed to react at 80°C for 4 h. After concentration under reduced pressure, it was purified by column chromatography (DCM:MeOH=10:1) to obtain 40 mg of 3-(1*H*-pyrazol-3-yl)-5-[4-(4-methylpiperazin-1-yl)phenyl]-1*H*-pyrrolo[2,3-*b*]pyridine with a yield of 69.4%. ¹H NMR (600 MHz, DMSO-d₆) δ 8.55 (s, 1H), 8.50 (s, 1H), 7.87 (s, 1H), 7.70 (s, 1H), 7.60 (d, *J =* 8.1 Hz, 2H), 7.48 (d, *J* = 7.6 Hz, 1H), 7.12 (d, *J =* 7.8 Hz, 1H), 7.07 (d, *J =* 8.2 Hz, 2H), 6.68 (s, 1H), 3.21 (s, 4H), 2.53 (s, 3H), 2.28 (d, *J =* 8.0 Hz, 4H).¹³C NMR (151 MHz, DMSO) δ 149.90, 147.75, 141.44, 137.45, 129.21, 128.53, 127.92, 127.27, 125.39, 123.81, 115.69, 101.27, 54.39, 47.81, 45.56.

### Example 3: Synthesis of compound 3, 3-(1Hindol-5-yl)-5-[4-(4-methylpiperazin-1-yl)phenyl]-1H-pyrrolo[2,3-b]pyridine

### 1) Synthesis of 3-(1H-indol-5-yl)-1-[(4-methylphenyl)dioxo-λ⁶-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 5-indoleboronic acid pinacol ester (1.5 eq., 0.39 mmol, 95 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 88 mg of 3-(1*H*-indol-5-yl)-1-[(4-methylphenyl)dioxo-λ⁶-thio]-5-[4-(4-methylpiperazin-1-yl)phenyl]pyrrolo[2,3-b] pyridine with a yield of 59.6%. ¹H NMR (600 MHz, Chloroform-*d*) δ 8.70 - 8.62 (m, 1H), 8.34 (s, 1H), 8.23 (d, *J* = 2.3 Hz, 1H), 8.13 (d, *J =* 8.2 Hz, 2H), 7.86 (d, *J =* 13.0 Hz, 2H), 7.50 (d, *J* = 9.2 Hz, 1H), 7.48 (d, *J* = 10.4 Hz, 2H), 7.43 (d, *J =* 8.4 Hz, 1H), 7.28 (d, *J =* 7.9 Hz, 2H), 7.00 (d, *J =* 8.3 Hz, 2H), 6.65 - 6.58 (m, 1H), 3.31 (d, *J =* 5.3 Hz, 4H), 2.68 (s, 4H), 2.42 (s, 3H), 2.37 (s, 3H).¹³C NMR (151 MHz, CDCl₃) δ 150.53, 146.73, 145.03, 143.87, 135.62, 135.38, 132.52, 129.67, 128.43, 128.18, 128.02, 126.65, 125.12, 124.32, 122.54, 122.28, 122.10, 121.83, 119.77, 116.37, 111.70, 102.94, 54.80, 48.49, 45.81, 21.65.

### 2) Synthesis of 3-(1H-indol-5-yl)-5-[4-(4-methylpiperazin-1-yl)phenyl]-1H-pyrrolo[2,3-b]pyridine

Into a 10 mL single-necked flask, 3-(1*H*-indol-5-yl)-1-[(4-methylphenyl)dioxo-λ⁶-thio]-5-[4-(4-methylpiperazin-1-yl)phenyl]pyrrolo[2,3-b]pyridine (1.0 eq., 0.16 mmol, 88 mg) and potassium carbonate (3.0 eq., 0.47 mmol, 65 mg) were added, and 5 mL of methanol was added. The mixture was allowed to react at 80°C for 4 h. After concentration under reduced pressure, it was purified by column chromatography (DCM:MeOH=10:1) to obtain 41 mg of 3-(1*H*-indol-5-yl)-5-[4-(4-methylpiperazin-1-yl)phenyl]-1*H*-pyrrolo[2,3-b]pyridine with a yield of 63.8%. ¹H NMR (600 MHz, DMSO-*d*₆) δ 8.49 (d, *J =* 2.1 Hz, 1H), 8.33 (d, *J =* 2.0 Hz, 1H), 7.88 (s, 1H), 7.73 (d, *J* = 2.5 Hz, 1H), 7.63 - 7.56 (m, 2H), 7.48 (s, 2H), 7.35 (t, *J* = 2.7 Hz, 1H), 7.09 - 7.02 (m, 2H), 6.48 (t, *J* = 2.5 Hz, 1H), 3.21 (d, *J =* 5.7 Hz, 4H), 2.61 - 2.51 (m, 4H), 2.28 (s, 3H).¹³C NMR (151 MHz, DMSO) δ 149.81, 148.09, 141.20, 134.54, 129.54, 128.38, 128.16, 127.43, 125.69, 125.49, 124.34, 122.98, 120.69, 117.65, 116.09, 115.76, 111.70, 101.12, 54.27, 47.74, 45.38.

### Example 4: Synthesis of compound 4, 3-(3-methyl-1H-pyrazol-4-yl)-5-[4-(4-methylpiperazin-1-yl)phenyl]-1H-pyrrolo[2,3-b]pyridine

### 1) Synthesis of 3-(3-methyl-1H-pyrazol-4-yl)-1-[(4-methylphenyl)dioxo-λ⁶-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-b]pyridine (1.0 eq., 0.26 mmol, 150 mg), 3-methylpyrazole-4-boronic acid pinacol ester (1.5 eq., 0.39 mmol, 82 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 82 mg of 3-(3-methyl-1*H*-pyrazol-4-yl)-1-[(4-methylphenyl)dioxo-λ⁶-thio]-5-[4-(4-methylpiperazin-1-yl)phenyl]pyrrolo[2,3-b]pyridine with a yield of 59.6%. ¹H NMR (600 MHz, DMSO-*d*₆) δ 8.65 (d, *J =* 2.2 Hz, 1H), 8.30 (s, 1H), 8.16 (s, 1H), 8.03 (d, *J* = 8.0 Hz, 2H), 7.83 (s, 1H), 7.62 (d, *J =* 8.2 Hz, 2H), 7.42 (d, *J =* 8.0 Hz, 2H), 7.03 (d, *J* = 8.3 Hz, 2H), 3.20 (t, *J* = 4.9 Hz, 4H), 2.48 (t, *J =* 4.8 Hz, 4H), 2.39 (s, 3H), 2.34 (s, 3H), 2.24 (s, 3H).¹³C NMR (151 MHz, DMSO) δ 150.45, 145.50, 145.39, 143.09, 134.55, 131.99, 129.91, 127.74, 127.35, 127.25, 125.94, 122.07, 121.76, 115.45, 112.69, 108.96, 54.34, 47.58, 45.57, 20.98.

### 2) Synthesis of 3-(3-methyl-1H-pyrazol-4-yl)-5-[4-(4-methylpiperazin-1-yl)phenyl]-1H-pyrrolo[2,3-b]pyridine

Into a 10 mL single-necked flask, 3-(3-methyl-1*H*-pyrazol-4-yl)-1-[(4-methylphenyl)dioxo-λ⁶-thio]-5-[4-(4-methylpiperazin-1-yl)phenyl]pyrrolo[2,3-b]pyridine (1.0 eq., 0.16 mmol, 82 mg) and potassium carbonate (3.0 eq., 0.47 mmol, 65 mg) were added, and 5 mL of methanol was added. The mixture was allowed to react at 80°C for 4 h. After concentration under reduced pressure, it was purified by column chromatography (DCM:MeOH=10:1) to obtain 38 mg of 3-(3-methyl-1*H-*pyrazol-4-yl)-5-[4-(4-methylpiperazin-1-yl)phenyl]-1*H*-pyrrolo[2,3-b]pyridine with a yield of 66.3%. ¹H NMR (600 MHz, DMSO-d₆) δ 8.48 (d, *J =* 2.2 Hz, 1H), 8.10 (s, 1H), 7.60 (d, *J =* 8.2 Hz, 2H), 7.48 (d, *J =* 6.8 Hz, 1H), 7.11 (d, *J* = 7.7 Hz, 1H), 7.03 (d, *J* = 8.3 Hz, 2H), 3.19 (t, *J =* 5.0 Hz, 4H), 2.34 (s, 3H), 2.26 (s, 3H).¹³C NMR (151 MHz, DMSO) δ 149.84, 147.54, 145.64, 141.25, 137.45, 129.35, 128.11, 127.91, 127.37, 125.38, 124.26, 122.89, 118.27, 115.67, 106.74, 54.38, 47.84, 45.54, 20.67.

### Example 5: Synthesis of compound 5, 3-(3,5-dimethyl-1H-pyrazol-4-yl)-5-[4-(4-methylpiperazin-1-yl)phenyl]-1H-pyrrolo[2,3-b]pyridine

### 1) Synthesis of 3-(3,5-dimethyl-1H-pyrazol-4-yl)-1-[(4-methylphenyl)dioxo-λ⁶-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 3,5-dimethylpyrazole-4-boronic acid pinacol ester (1.5 eq., 0.39 mmol, 87 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 90 mg of 3-(3,5-dimethyl-1*H*-pyrazol-4-yl)-1-[(4-methylphenyl)dioxo-λ⁶-thio]-5-[4-(4-methylpiperazin-1-yl)phenyl]pyrrolo[2,3-b]pyridine with a yield of 63.8%. ¹H NMR (600 MHz, Chloroform-*d*) δ 8.65 (s, 1H), 8.13 (d, *J =* 8.0 Hz, 2H), 7.73 (s, 1H), 7.60 (s, 1H), 7.44 (d, *J =* 8.1 Hz, 2H), 7.31 (d, *J =* 8.1 Hz, 2H), 6.99 (d, *J =* 8.3 Hz, 2H), 3.40 - 3.25 (m, 4H), 2.73 (s, 4H), 2.46 (s, 3H), 2.39 (s, 3H), 2.23 (s, 6H).¹³C NMR (151 MHz, CDCl₃) δ 150.46, 146.32, 145.22, 143.97, 135.48, 132.40, 129.73, 129.53, 128.09, 128.07, 126.35, 124.61, 123.15, 116.45, 111.77, 108.79, 58.48, 54.74, 48.31, 45.68, 21.69.

### 2) Synthesis of 3-(3,5-dimethyl-1H-pyrazol-4-yl)-5-[4-(4-methylpiperazin-1-yl)phenyl]-1H-pyrrolo[2,3-b]pyridine

Into a 10 mL single-necked flask, 3-(3,5-dimethyl-1*H*-pyrazol-4-yl)-1-[(4-methylphenyl)dioxo-λ⁶-thio]-5-[4-(4-methylpiperazin-1-yl)phenyl]pyrrolo[2,3-b]pyridine (1.0 eq., 0.17 mmol, 90 mg) and potassium carbonate (3.0 eq., 0.50 mmol, 69 mg) were added, and 5 mL of methanol was added. The mixture was allowed to react at 80°C for 4 h. After concentration under reduced pressure, it was purified by column chromatography (DCM:MeOH=10:1) to obtain 42 mg of 3-(3,5-dimethyl-1*H*-pyrazol-4-yl)-5-[4-(4-methylpiperazin-1-yl)phenyl]-1*H-*pyrrolo[2,3-b]pyridine with a yield of 65.2%. ¹H NMR (600 MHz, Methanol-d₄) δ 8.44 (d, *J =* 2.4 Hz, 1H), 7.88 (t, *J =* 2.4 Hz, 1H), 7.71 (dd, *J =* 8.2, 2.3 Hz, 2H), 7.55 (dd, *J =* 8.9, 2.4 Hz, 2H), 7.35 (d, *J =* 2.4 Hz, 1H), 7.24 (dd, *J =* 8.3, 2.2 Hz, 2H), 7.11 (dd, *J =* 8.9, 2.4 Hz, 2H), 3.45 (s, 3H), 3.21 (qd, *J =* 7.4, 2.4 Hz, 3H), 2.87 (d, *J =* 2.3 Hz, 3H), 2.36 (d, *J =* 2.3 Hz, 3H), 2.21 (d, *J =* 2.3 Hz, 6H).¹³C NMR (151 MHz, DMSO) δ 148.39, 147.72, 145.57, 141.08, 137.51, 130.47, 127.94, 127.71, 127.40, 125.38, 124.10, 119.04, 116.33, 109.52, 54.81, 52.13, 45.51, 45.48.

### Example 6: Synthesis of compound 6, 5-[4-(4-methylpiperazin-1-yl)phenyl]-3-(2-methylpyrazol-3-yl)-1H-pyrrolo[2,3-b]pyridine

### 1) Synthesis of 1-[(4-methylphenyl)dioxo-λ⁶-thio]-5-[4-(4-methylpiperazin-1-yl)phenyl]-3-(2-methylpyrazol-3-yl)pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 1-methyl-1*H-*pyrazole-5-boronic acid pinacol ester (1.5 eq., 0.39 mmol, 82 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 86 mg of 1-[(4-methylphenyl)dioxo-λ⁶-thio]-5-[4-(4-methylpiperazin-1-yl)phenyl]-3-(2-methylpyrazol-3-yl)pyrrolo[2,3-b]pyridine with a yield of 62.2%. ¹H NMR (600 MHz, Chloroform-*d*) δ 8.70 (d, *J =* 2.8 Hz, 1H), 8.20 - 8.13 (m, 2H), 7.93 (d, *J* = 2.6 Hz, 1H), 7.82 (d, *J* = 2.3 Hz, 1H), 7.62 - 7.59 (m, 1H), 7.46 (dt, *J =* 8.6, 4.0 Hz, 2H), 7.32 (d, *J* = 8.1 Hz, 2H), 7.04 - 6.96 (m, 2H), 6.47 - 6.41 (m, 1H), 3.94 (d, *J* = 2.3 Hz, 3H), 3.32 (s, 4H), 2.68 (s, 4H), 2.42 (s, 3H), 2.40 (d, *J* = 2.2 Hz, 3H).¹³C NMR (151 MHz, CDCl₃) δ 150.75, 145.82, 145.62, 144.71, 138.99, 135.09, 134.11, 133.05, 129.82, 128.26, 128.09, 126.22, 124.79, 121.90, 116.32, 109.22, 107.80, 106.99, 54.82, 48.41, 45.87, 37.62, 37.28.

### 2) Synthesis of 5-[4-(4-methylpiperazin-1-yl)phenyl]-3-(2-methylpyrazol-3-yl)-1H-pyrrolo[2,3-b]pyridine

Into a 10 mL single-necked flask, 1-[(4-methylphenyl)dioxo-λ⁶-thio]-5-[4-(4-methylpiperazin-1-yl)phenyl]-3-(2-methylpyrazol-3-yl)pyrrolo[2,3-b]pyridine (1.0 eq., 0.16 mmol, 86 mg) and potassium carbonate (3.0 eq., 0.49 mmol, 68 mg) were added, and 5 mL of methanol was added. The mixture was allowed to react at 80°C for 4 h. After concentration under reduced pressure, it was purified by column chromatography (DCM:MeOH=10:1) to obtain 41 mg of 5-[4-(4-methylpiperazin-1-yl)phenyl]-3-(2-methylpyrazol-3-yl)-1*H*-pyrrolo[2,3-*b*]pyridine with a yield of 66.9%. ¹H NMR (600 MHz, Chloroform-d) δ 8.62 (d, *J =* 2.0 Hz, 1H), 8.08 (d, *J =* 2.0 Hz, 1H), 7.61 (d, *J =* 1.8 Hz, 1H), 7.54 (s, 1H), 7.53 (s, 1H), 7.49 (s, 1H), 7.05 (s, 1H), 7.03 (s, 1H), 6.45 (d, *J =* 1.8 Hz, 1H), 3.95 (s, 3H), 3.31 (t, *J =* 5.0 Hz, 4H), 2.65 (t, *J =* 4.9 Hz, 4H).¹³C NMR (151 MHz, CDCl₃) δ 150.55, 147.59, 142.86, 138.83, 136.36, 130.56, 130.09, 128.06, 125.98, 124.55, 119.35, 116.38, 106.32, 105.27, 54.97, 48.78, 46.04, 37.44.

### Example 7: Synthesis of compound 7, 3-(2-ethylpyrazol-3-yl)-5-[4-(4-methylpiperazin-1-yl)phenyl]-1H-pyrrolo[2,3-b]pyridine

### 1) Synthesis of 3-(2-ethylpyrazol-3-yl)-1-[(4-methylphenyl)dioxo-λ6-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 1-ethylpyrazole-5-boronic acid pinacol ester (1.5 eq., 0.39 mmol, 87 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 87 mg of 3-(2-ethylpyrazol-3-yl)-1-[(4-methylphenyl)dioxo-λ⁶-thio]-5-[4-(4-methylpiperazin-1-yl)phenyl]pyrrolo[2,3-b]pyridine with a yield of 61.7%. ¹H NMR (600 MHz, Chloroform-*d*) δ 8.68 (s, 1H), 8.16 (d, *J =* 7.8 Hz, 2H), 7.90 (s, 1H), 7.81 (s, 1H), 7.63 (s, 1H), 7.48 (s, 2H), 7.33 (d, *J =* 7.9 Hz, 2H), 7.00 (s, 2H), 6.40 (s, 1H), 4.32 - 4.14 (m, 2H), 3.72 (s, 4H), 3.07 (s, 3H), 2.89 (s, 3H), 2.40 (s, 4H), 1.45 (d, *J* = 7.9 Hz, 3H).¹³C NMR (151 MHz, CDCl₃) δ 146.01, 145.70, 144.71, 139.19, 135.04, 132.55, 131.19, 129.86, 128.42, 128.29, 126.36, 124.93, 117.80, 109.28, 107.21, 53.93, 46.87, 44.80, 21.73, 15.83.

### 2) Synthesis of 3-(2-ethylpyrazol-3-yl)-5-[4-(4-methylpiperazin-1-yl)phenyl]-1H-pyrrolo[2,3-b]pyridine

Into a 10 mL single-necked flask, 3-(2-ethylpyrazol-3-yl)-1-[(4-methylphenyl)dioxo-λ⁶-thio]-5-[4-(4-methylpiperazin-1-yl)phenyl]pyrrolo[2,3-b]pyridine (1.0 eq., 0.16 mmol, 87 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 40 mg of 3-(2-ethylpyrazol-3-yl)-5-[4-(4-methylpiperazin-1-yl)phenyl]-1*H*-pyrrolo[2,3-*b*]pyridine with a yield of 64.7%. ¹H NMR (600 MHz, DMSO-d₆) δ 8.56 (d, *J =* 2.1 Hz, 1H), 8.01 (d, *J =* 2.1 Hz, 1H), 7.76 (d, *J =* 2.5 Hz, 1H), 7.59 (d, *J =* 8.6 Hz, 2H), 7.55 (d, *J =* 1.8 Hz, 1H), 7.04 (d, *J =* 8.4 Hz, 2H), 6.52 (d, *J =* 1.8 Hz, 1H), 4.21 (q, *J =* 7.2 Hz, 2H), 3.23 (s, 4H), 2.63 (s, 4H), 2.34 (s, 3H), 1.33 (t, *J =* 7.2 Hz, 3H).¹³C NMR (151 MHz, DMSO) δ 150.35, 147.98, 142.61, 138.75, 135.72, 129.70, 129.48, 128.02, 126.16, 124.48, 118.86, 116.34, 106.11, 103.95, 54.58, 47.97, 44.41, 15.98.

### Example 8: Synthesis of compound 8, 5-[4-(4-methylpiperazin-1-yl)phenyl]-3-[2-(prop-2-yl)pyrazol-3-yl]-1H-pyrrolo[2,3-b]pyridine

### 1) Synthesis of 1-[(4-methylphenyl)dioxo-λ⁶-thio]-5-[4-(4-methylpiperazin-1-yl)phenyl]-3-[2-(prop-2-yl)pyrazol-3-yl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 1-isopropylpyrazole-5-boronic acid pinacol ester (1.5 eq., 0.39 mmol, 93 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 91 mg of 1-[(4-methylphenyl)dioxo-λ⁶-thio]-5-[4-(4-methylpiperazin-1-yl)phenyl]-3-[2-(prop-2-yl)pyrazol-3-yl]pyrrolo[2,3-b]pyridine with a yield of 62.5%. ¹H NMR (600 MHz, Chloroform-d) δ 8.69 (s, 1H), 8.16 (d, *J =* 8.0 Hz, 2H), 7.87 (s, 1H), 7.77 (s, 1H), 7.65 (s, 1H), 7.44 (d, *J* = 8.3 Hz, 2H), 7.33 (d, *J* = 8.0 Hz, 2H), 6.99 (d, *J =* 8.2 Hz, 2H), 6.35 (s, 1H), 4.56 (p, *J =* 6.7 Hz, 1H), 3.31 (t, *J =* 5.1 Hz, 4H), 2.67 (s, 4H), 2.42 (s, 3H), 2.40 (s, 3H), 1.50 (d, *J =* 6.5 Hz, 6H).¹³C NMR (151 MHz, CDCl₃) δ 150.72, 145.82, 145.59, 144.65, 139.10, 135.12, 133.02, 132.51, 129.81, 128.90, 128.28, 128.03, 125.94, 124.86, 122.41, 116.31, 109.36, 106.92, 54.82, 50.43, 48.40, 45.86, 23.02, 21.71.

### 2) Synthesis of 5-[4-(4-methylpiperazin-1-yl)phenyl]-3-[2-(prop-2-yl)pyrazol-3-yl]-1H-pyrrolo[2,3-b]pyridine

Into a 10 mL single-necked flask, 1-[(4-methylphenyl)dioxo-λ⁶-thio]-5-[4-(4-methylpiperazin-1-yl)phenyl]-3-[2-(prop-2-yl)pyrazol-3-yl]pyrrolo[2,3-b]pyridine (1.0 eq., 0.16 mmol, 91 mg) and potassium carbonate (3.0 eq., 0.49 mmol, 68 mg) were added, and 5 mL of methanol was added. The mixture was allowed to react at 80°C for 4 h. After concentration under reduced pressure, it was purified by column chromatography (DCM:MeOH=10:1) to obtain 44 mg of 5-[4-(4-methylpiperazin-1-yl)phenyl]-3-[2-(prop-2-yl)pyrazol-3-yl]-1*H*-pyrrolo[2,3-*b*]pyridine with a yield of 66.4%. ¹H NMR (600 MHz, DMSO-d₆) δ 8.56 (d, *J =* 2.1 Hz, 1H), 7.95 (d, *J =* 2.1 Hz, 1H), 7.70 (d, *J =* 2.6 Hz, 1H), 7.58 (d, *J =* 2.0 Hz, 1H), 7.57 (d, *J =* 2.0 Hz, 2H), 7.08 - 6.99 (m, 2H), 6.44 (d, *J =* 1.7 Hz, 1H), 4.66 (p, *J* = 6.5 Hz, 1H), 3.23 (s, 4H), 2.60 (s, 4H), 2.33 (s, 3H), 1.39 (d, *J* = 6.5 Hz, 6H).¹³C NMR (151 MHz, DMSO) δ 149.78, 147.39, 142.00, 138.14, 134.58, 128.97, 128.88, 127.36, 125.75, 123.63, 118.53, 115.73, 105.51, 103.33, 54.06, 49.21, 47.42, 45.07, 22.82.

### Example 9: Synthesis of compound 9, 5-[4-(4-methylpiperazin-1-yl)phenyl]-3-(1-methylpyrazol-4-yl)-1H-pyrrolo[2,3-b]pyridine

### 1) Synthesis of 1-[(4-methylphenyl)dioxo-λ⁶-thio]-5-[4-(4-methylpiperazin-1-yl)phenyl]-3-(1-methylpyrazol-4-yl)pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 1-methyl-4-(4,4,5,5-tetramethyl-1,3,2-dioxopentaboran-2-yl)-1*H*-pyrazole (1.5 eq., 0.39 mmol, 82 mg), potassium carbonate (3.0 eq., 0.79 mmol, 109 mg), [1,1'-bis(diphenylphosphino)ferrocene]dichloropalladium (II) (0.05 eq., 0.01 mmol, 10 mg)were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 84 mg of 1-[(4-methylphenyl)dioxo-λ⁶-thio]-5-[4-(4-methylpiperazin-1-yl)phenyl]-3-(1-methylpyrazol-4-yl)pyrrolo[2,3-b]pyridine with a yield of 60.7%. ¹H NMR (600 MHz, Chloroform-*d*) δ 8.65 (d, *J =* 2.4 Hz, 1H), 8.10 (dd, *J* = 8.3, 2.3 Hz, 2H), 8.03 (d, *J* = 2.6 Hz, 1H), 7.77 (t, *J =* 3.1 Hz, 2H), 7.65 (d, *J =* 2.3 Hz, 1H), 7.52 - 7.44 (m, 2H), 7.28 (d, *J =* 8.6 Hz, 2H), 7.05 - 6.97 (m, 2H), 3.99 (d, *J =* 2.3 Hz, 3H), 3.37 (s, 4H), 2.75 (s, 3H), 2.48 (s, 3H), 2.37 (d, *J* = 2.3 Hz, 3H).¹³C NMR (151 MHz, CDCl₃) δ 146.42, 145.16, 144.11, 137.50, 135.43, 132.52, 129.68, 128.17, 128.02, 127.50, 126.10, 121.97, 121.82, 116.51, 113.65, 111.81, 58.47, 54.67, 48.27, 39.18, 21.64.

### 2) Synthesis of 5-[4-(4-methylpiperazin-1-yl)phenyl]-3-(1-methylpyrazol-4-yl)-1H-pyrrolo[2,3-b]pyridine

Into a 10 mL single-necked flask, 1-[(4-methylphenyl)dioxo-λ⁶-thio]-5-[4-(4-methylpiperazin-1-yl)phenyl]-3-(1-methylpyrazol-4-yl)pyrrolo[2,3-b]pyridine (1.0 eq., 0.16 mmol, 84 mg) and potassium carbonate (3.0 eq., 0.48 mmol, 66 mg) were added, and 5 mL of methanol was added. The mixture was allowed to react at 80°C for 4 h. After concentration under reduced pressure, it was purified by column chromatography (DCM:MeOH=10:1) to obtain 39 mg of 5-[4-(4-methylpiperazin-1-yl)phenyl]-3-(1-methylpyrazol-4-yl)-1*H*-pyrrolo[2,3-b]pyridine with a yield of 65.3%. ¹H NMR (600 MHz, DMSO-d₆) δ 8.47 (s, 1H), 8.26 (s, 1H), 8.22 (s, 1H), 7.86 (s, 1H), 7.69 (d, *J =* 2.8 Hz, 1H), 7.64 (d, *J =* 8.2 Hz, 2H), 7.06 (d, *J =* 8.2 Hz, 2H), 3.90 (s, 3H), 3.22 (s, 4H), 2.57 (s, 4H), 2.30 (s, 3H). ¹³C NMR (151 MHz, DMSO) δ 149.78, 147.71, 141.25, 135.96, 129.39, 128.22, 127.48, 126.86, 124.24, 122.30, 117.26, 115.70, 115.17, 106.48, 54.20, 47.69, 45.30, 38.38.

### Example 10: Synthesis of compound 10, 3-(1H-pyrazol-3-yl)-5-(4-[[2-(1,4-oxazacyclohexan-4-yl)ethyl]oxy]phenyl)-1H-pyrrolo[2,3-b]pyridine:

### 1) Synthesis of 3-(1H-pyrazol-3-yl)-1-[(4-methylphenyl)dioxo-λ⁶-thio]-5-(4-[[2-(1,4-oxazacyclohexan-4-yl)ethyl]oxy]phenyl)pyrrolo[2,3-b]pyridine

Into a 50 mL two-necked flask, 3-iodo-1-[(4-methylphenyl)dioxo-λ⁶-thio]-5-(4-{[2-(1,4-oxazacyclohexan-4-yl)ethyl]oxy}phenyl)pyrrolo[2,3-b] pyridine (1.0 eq., 0.26 mmol, 150 mg), 1*H*-pyrazole-3-boronic acid pinacol ester (1.5 eq., 0.39 mmol, 76 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 83 mg of 3-(1*H*-pyrazol-3-yl)-1-[(4-methylphenyl)dioxo-λ⁶-thio]-5-(4-[[2-(1,4-oxazacyclohexan-4-yl)ethyl]oxy]phenyl)pyrrolo[2,3-b]pyridine with a yield of 62.4%. ¹H NMR (600 MHz, Chloroform-d) δ 8.64 (d, *J* = 2.4 Hz, 1H), 8.58 (s, 1H), 8.11 (dd, *J* = 8.5, 2.0 Hz, 2H), 8.03 (s, 1H), 7.66 (s, 1H), 7.53 - 7.47 (m, 2H), 7.28 (s, 2H), 7.00 - 6.95 (m, 2H), 6.64 (s, 1H), 4.20 (d, *J =* 5.9 Hz, 2H), 3.79 (d, *J =* 5.2 Hz, 4H), 2.89 (s, 2H), 2.67 (s, 4H), 2.36 (d, *J =* 1.9 Hz, 3H). ¹³C NMR (151 MHz, CDCl₃) δ 158.36, 146.60, 145.25, 144.27, 143.57, 135.34, 132.61, 131.24, 129.70, 128.56, 128.49, 128.05, 124.82, 123.46, 121.22, 115.10, 113.17, 103.13, 66.66, 65.62, 57.58, 54.01, 21.64.

### 2) Synthesis of 3-(1H-pyrazol-3-yl)-5-(4-[[2-(1,4-oxazacyclohexan-4-yl)ethyl]oxy]phenyl)-1H-pyrrolo[2,3-b]pyridine

Into a 10 mL single-necked flask, 3-(1*H*-pyrazol-3-yl)-1-[(4-methylphenyl)dioxo-λ⁶-thio]-5-(4-[[2-(1,4-oxazacyclohexan-4-yl)ethyl]oxy]phenyl)pyrrolo[2,3-b]pyridine (1.0 eq., 0.16 mmol, 83 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 24 mg of 3-(1*H*-pyrazol-3-yl)-5-(4-[[2-(1,4-oxazacyclohexan-4-yl)ethyl]oxy]phenyl)-1*H*-pyrrolo[2,3-b]pyridine with a yield of 59.4%. ¹H NMR (600 MHz, Methanol-d₄) δ 7.64 (s, 1H), 7.56 (d, *J* = 2.1 Hz, 1H), 6.87 (s, 1H), 6.79 (d, *J =* 2.2 Hz, 1H), 6.77 - 6.70 (m, 2H), 6.18 (d, *J =* 8.6 Hz, 2H), 5.77 (d, *J =* 2.2 Hz, 1H), 3.31 (t, *J =* 5.4 Hz, 2H), 2.84 (t, *J =* 4.6 Hz, 4H), 1.96 (t, *J =* 5.4 Hz, 2H), 1.75 (t, *J =* 4.6 Hz, 4H). ¹³C NMR (151 MHz, DMSO) δ 157.65, 147.87, 141.59, 131.35, 128.30, 127.91, 125.99, 123.92, 117.02, 114.98, 101.30, 66.09, 65.32, 56.93, 53.55.

### Example 11: Synthesis of compound 11, 5-(4-[[2-(hexahydropyridin-1-yl)ethyl]oxy]phenyl)-3-(1H-pyrazol-3-yl)-1H-pyrrolo[2,3-b]pyridine:

### 1) Synthesis of 5-(4-[[2-(hexahydropyridin-1-yl)ethyl]oxy]phenyl)3-(1H-pyrazol-3-yl)-1-[(4-methylphenyl)dioxo-λ⁶-thio]pyrrolo[2,3-b]pyridine

Into a 50 mL two-necked flask, 5-(4-{[2-(hexahydropyridin-1-yl)ethyl]oxy}phenyl)-3-iodo-1-[(4-methylphenyl)dioxo-λ⁶-thio]pyrrolo[2,3-b]pyridine (1.0 eq., 0.26 mmol, 150 mg), 1*H-*pyrazole-3-boronic acid pinacol ester (1.5 eq., 0.39 mmol, 76 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 65 mg of 5-(4-[[2-(hexahydropyridin-1-yl)ethyl]oxy]phenyl)3-(1*H*-pyrazol-3-yl)-1-[(4-methylphenyl)dioxo-λ⁶-thio]pyrrolo[2,3-b]pyridine with a yield of 42.3%. ¹H NMR (600 MHz, Chloroform-*d*) δ 8.62 (d, *J* = 2.1 Hz, 1H), 8.57 (s, 1H), 8.10 (d, *J =* 8.1 Hz, 2H), 8.02 (s, 1H), 7.66 (d, *J =* 2.4 Hz, 1H), 7.47 (d, *J =* 8.3 Hz, 2H), 7.27 (s, 1H), 6.94 (d, *J =* 8.4 Hz, 2H), 6.63 (d, *J =* 2.3 Hz, 1H), 4.23 (t, *J =* 5.6 Hz, 2H), 3.00 (t, *J =* 5.5 Hz, 2H), 2.75 (s, 4H), 2.36 (s, 3H), 1.72 (p, *J =* 5.6 Hz, 4H), 1.52 (s, 2H). ¹³C NMR (151 MHz, CDCl₃) δ 158.13, 146.59, 145.26, 144.24, 135.31, 132.57, 131.28, 129.70, 128.55, 128.03, 123.39, 121.24, 115.05, 103.05, 65.06, 57.37, 54.72, 25.01, 23.51, 21.64.

### 2) Synthesis of 5-(4-[[2-(hexahydropyridin-1-yl)ethyl]oxy]phenyl)-3-(1H-pyrazol-3-yl)-1H-pyrrolo[2,3-b]pyridine

Into a 10 mL single-necked flask, 5-(4-[[2-(hexahydropyridin-1-yl)ethyl]oxy]phenyl)3-(1*H-*pyrazol-3-yl)-1-[(4-methylphenyl)dioxo-λ⁶-thio]pyrrolo[2,3-b]pyridine (1.0 eq., 0.16 mmol, 83 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 32 mg of 5-(4-[[2-(hexahydropyridin-1-yl)ethyl]oxy]phenyl)-3-(1*H*-pyrazol-3-yl)-1*H*-pyrrolo[2,3-b]pyridine with a yield of 53.4%. ¹H NMR (600 MHz, DMSO-*d*₆) δ 8.56 (s, 1H), 8.51 (s, 1H), 7.88 (s, 1H), 7.70 (s, 1H), 7.68 - 7.57 (m, 2H), 7.07 (d, *J =* 8.1 Hz, 2H), 6.68 (s, 1H), 4.12 (t, *J =* 6.1 Hz, 2H), 2.69 (d, *J =* 7.8 Hz, 2H), 2.46 (s, 4H), 1.51 (p, *J =* 5.6 Hz, 4H), 1.39 (q, *J =* 6.1, 5.7 Hz, 2H). ¹³C NMR (151 MHz, DMSO) δ 157.69, 147.86, 141.56, 131.29, 128.30, 127.89, 123.91, 114.97, 101.30, 65.57, 57.26, 54.31, 25.47, 23.82.

### Example 12: Synthesis of compound 12, 3-(2-methylpyrazol-3-yl)-5-(4-{[2-(1,4-oxazacyclohexan-4-yl)ethyl]oxy}phenyl)-1H-pyrrolo[2,3-b]pyridine

### 1) Synthesis of 5-bromo-3-iodo-1H-pyrrolo[2,3-b]pyridine:

Into a 50 mL two-necked flask, 5-bromo-1*H*-pyrrolo[2,3-b]pyridine (1.0 eq., 25.38 mmol, 5.00 g) was added, then 60 mL of anhydrous THF was added, N-iodosuccinimide (1.1 eq., 27.91 mmol, 6.28 g) was slowly added in batches, and the mixture was allowed to react for 60 mins. After the reaction stopped, saturated sodium thiosulfate solution was added to quench the reaction, and then a large amount of water was added to precipitate the product. The product was directly filtered without other post-treatment to obtain 7.57 g of crude product 5-bromo-3-iodo-1*H*-pyrrolo[2,3-b]pyridine with a yield of 92.4%.

### 2) Synthesis of 5-bromo-3-iodo-1-[(4-methylphenyl)dioxo-λ⁶-thio]pyrrolo[2,3-b]pyridine:

Into a 50 mL two-necked flask, 5-bromo-3-iodo-1*H*-pyrrolo[2,3-b]pyridine (1.0 eq., 18.58 mmol, 6.00 g) and 40 mL of anhydrous THF were added. In ice bath, sodium hydride (60%, dispersed in liquid paraffin) (1.1 eq., 20.44 mmol, 817 mg) was slowly added and the mixture was allowed to react for 30 mins. Then, p-toluenesulfonyl chloride (1.3 eq., 24.15 mmol, 4.60 g) was slowly added and the reaction was carried out at room temperature for 4 h. After the reaction was completed, ice water was added to quench the reaction. The product precipitated and was directly filtered to obtain a crude product, which was recrystallized to obtain 6.43 g of 5-bromo-3-iodo-1-[(4-methylphenyl)dioxo-λ⁶-thio]pyrrolo[2,3-b]pyridine with a yield of 72.5%.

### 3) Synthesis of 5-bromo-1-[(4-methylphenyl)dioxo-λ⁶-thio]-3-(2-methylpyrazol-3-yl)pyrrolo[2,3-b]pyridine:

Into a 50 mL two-necked flask, 5-bromo-3-iodo-1-[(4-methylphenyl)dioxo-λ⁶-thio]pyrrolo[2,3-b]pyridine (1.0 eq., 12.58 mmol, 6.00 g), 1-methyl-1*H*-pyrazole-5-boronic acid pinacol ester (1.5 eq., 18.86 mmol, 3.92 g), potassium carbonate (3.0 eq., 37.73 mmol, 5.21 g), [1,1'-bis(diphenylphosphino)ferrocene]dichloropalladium (II) (0.05 eq., 0.63 mmol, 456 mg) were added, and then 45 mL of 1,4-dioxane and 15 mL of water were added. The atmosphere was replaced with argon three times and the mixture was heated at 80°C to react for 72 h. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 2.93 g of 5-bromo-1-[(4-methylphenyl)dioxo-λ⁶-thio]-3-(2-methylpyrazol-3-yl)pyrrolo[2,3-b]pyridine with a yield of 54.1%

### 4) Synthesis of 1-[(4-methylphenyl)dioxo-λ⁶-thio]-3-(2-methylpyrazol-3-yl)-5-(4-{[2-(1,4-oxazacyclohexan-4-yl)ethyl]oxy}phenyl)pyrrolo[2,3-b]pyridine

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-(2-morpholinethoxy)phenylboronic acid pinacol ester (1.5 eq., 0.35 mmol, 116 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 84 mg of 1-[(4-methylphenyl)dioxo-^{λ}6-thio]-3-(2-methylpyrazol-3-yl)-5-(4-{[2-(1,4-oxazacyclohexan-4-yl)ethyl]oxy}phenyl)pyrrolo [2,3-b]pyridine with a yield of 65.1%. ¹H NMR (600 MHz, Chloroform-d) δ 8.68 (s, 1H), 8.16 (dd, *J =* 8.4, 2.1 Hz, 2H), 7.93 (s, 1H), 7.84 (d, *J* = 2.1 Hz, 1H), 7.60 (s, 1H), 7.46 (dd, *J =* 8.6, 2.1 Hz, 2H), 7.33 (d, *J =* 8.1 Hz, 2H), 7.00 (dd, *J =* 8.6, 2.2 Hz, 2H), 6.44 (s, 1H), 4.21 (s, 2H), 3.94 (d, *J* = 2.0 Hz, 3H), 3.79 (s, 4H), 2.96 - 2.81 (m, 2H), 2.66 (s, 4H), 2.40 (s, 3H).¹³C NMR (151 MHz, CDCl₃) δ 145.95, 145.66, 144.79, 139.00, 135.05, 134.02, 132.89, 130.75, 129.83, 128.54, 128.28, 126.55, 124.89, 121.89, 115.27, 109.20, 107.00, 57.52, 53.98, 37.62, 21.71.

### 5) Synthesis of 3-(2-methylpyrazol-3-yl)-5-(4-{[2-(1,4-oxazacyclohexan-4-yl)ethyl]oxy} phenyl)-1H-pyrrolo[2,3-b]pyridine

Into a 10 mL single-necked flask, 1-[(4-methylphenyl)dioxo-λ⁶-thio]-3-(2-methylpyrazol-3-yl)-5-(4-{[2-(1,4-oxazacyclohexan-4-yl)ethyl]oxy}phenyl)pyrrolo[2,3-b]pyridine (1.0 eq., 0.17 mmol, 84 mg) and potassium carbonate (3.0 eq., 0.51 mmol, 70 mg) were added, and 5 mL of methanol was added. The mixture was allowed to react at 80°C for 4 h. After concentration under reduced pressure, it was purified by column chromatography (DCM:MeOH=10:1) to obtain 28 mg of 3-(2-methylpyrazol-3-yl)-5-(4-{[2-(1,4-oxazacyclohexan-4-yl)ethyl]oxy}phenyl)-1*H*-pyrrolo [2,3-b]pyridine with a yield of 45.3%. ¹H NMR (600 MHz, DMSO-*d*₆) δ 7.72 (d, *J =* 2.3 Hz, 1H), 7.27 (d, *J =* 2.4 Hz, 1H), 7.02 (d, *J =* 2.7 Hz, 1H), 6.86 - 6.80 (m, 2H), 6.67 (t, *J =* 1.8 Hz, 1H), 6.24 - 6.17 (m, 2H), 5.76 (t, *J =* 1.7 Hz, 1H), 3.35 - 3.27 (m, 2H), 3.11 - 3.04 (m, 3H), 2.75 (q, *J* = 3.6, 2.6 Hz, 4H), 1.88 (t, *J =* 5.8 Hz, 2H), 1.65 (s, 4H).¹³C NMR (151 MHz, DMSO) δ 157.85, 147.61, 142.25, 137.98, 135.94, 131.15, 128.82, 128.19, 125.96, 124.61, 118.11, 115.05, 105.24, 103.65, 66.17, 65.42, 57.01, 53.63, 37.41.

### Example 13: Synthesis of compound 13, 5-(4-{[2-(hexahydropyridin-1-yl)ethyl]oxy}phenyl)-3-(2-methylpyrazol-3-yl)-1H-pyrrolo[2,3-b]pyridine

### 1) Synthesis of 5-(4-{[2-(hexahydropyridin-1-yl)ethyl]oxy}phenyl)-1-[(4-methylphenyl)dioxo-λ⁶-thio]-3-(2-methylpyrazol-3-yl)pyrrolo[2,3-b]pyridine

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-(2-(piperidin-1-yl)ethoxy)phenylboronic acid pinacol ester (1.5 eq., 0.35 mmol, 115 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 80 mg of 5-(4-{[2-(hexahydropyridin-1-yl)ethyl]oxy}phenyl)-1-[(4-methylphenyl)dioxo-λ⁶-thio]-3-(2-methylpyrazol-3-yl)pyrrolo[2,3-b]pyridine with a yield of 62.1%. ¹H NMR (600 MHz, Chloroform-*d*) δ 8.87 (s, 1H), 8.21 (s, 1H), 7.69 (d, *J* = 8.2 Hz, 2H), 7.61 (d, *J* = 2.3 Hz, 1H), 7.74 (d, *J* = 8.1 Hz, 2H), 7.53 (s, 1H), 7.26 (d, *J* = 8.2 Hz, 2H),7.01 (d, *J =* 8.1 Hz, 2H), 6.45 (d, *J =* 2.4 Hz, 1H), 4.27 (s, 2H), 3.95 (d, *J* = 2.3 Hz, 3H), 3.06 (s, 2H), 2.69 (s, 4H), 2.37 (s, 3H), 1.84 (s, 4H), 1.52 (s, 2H).

### 2) Synthesis of 5-(4-{[2-(hexahydropyridin-1-yl)ethyl]oxy}phenyl)-3-(2-methylpyrazol-3-yl)-1H-pyrrolo[2,3-b]pyridine

Into a 10 mL single-necked flask, 5-(4-{[2-(hexahydropyridin-1-yl)ethyl]oxy}phenyl)-1-[(4-methylphenyl)dioxo-λ⁶-thio]3-(2-methylpyrazol-3-yl)pyrrolo[2,3-b]pyridine (1.0 eq., 0.14 mmol, 80 mg) and potassium carbonate (3.0 eq., 0.43 mmol, 60 mg) were added, and 5 mL of methanol was added. The mixture was allowed to react at 80°C for 4 h. After concentration under reduced pressure, it was purified by column chromatography (DCM:MeOH=10:1) to obtain 27 mg of 5-(4-{[2-(hexahydropyridin-1-yl)ethyl]oxy}phenyl)-3-(2-methylpyrazol-3-yl)-1*H*-pyrrolo[2,3-b]pyridine with a yield of 47.2%. ¹H NMR (600 MHz, Chloroform-d) δ 8.60 (s, 1H), 8.06 (s, 1H), 7.61 (d, *J =* 2.3 Hz, 1H), 7.53 (d, *J =* 8.1 Hz, 2H), 7.48 (s, 1H), 7.01 (d, *J* = 8.1 Hz, 2H), 6.45 (d, *J* = 2.4 Hz, 1H), 4.27 (s, 2H), 3.95 (d, *J =* 2.3 Hz, 3H), 2.96 (s, 2H), 2.69 (s, 4H), 1.73 (s, 4H), 1.51 (s, 2H).¹³C NMR (151 MHz, CDCl₃) δ 147.64, 143.27, 138.85, 136.19, 130.47, 128.51, 126.12, 124.82, 124.39, 119.16, 115.17, 106.36, 105.49, 58.47, 57.58, 54.87, 37.44.

### Example 14: Synthesis of compound 14, (4-methylpiperazin-1-yl){4-[3-(2-methylpyrazol-3-yl)-1H-pyrrolo[2,3-b]pyridin-5-yl]phenyl}ketone

### 1) Synthesis of (4-{1-[(4-methylphenyl)dioxo-λ⁶-thio]-3-(2-methylpyrazol-3-yl)pyrrolo[2,3-b]pyridin-5-yl}phenyl)(4-methylpiperazin-1-yl)ketone

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-(4-methylpiperazin-1-carbonyl)phenylboronic acid pinacol ester (1.5 eq., 0.35 mmol, 115 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 82 mg of (4-{1-[(4-methylphenyl)dioxo-λ⁶-thio]-3-(2-methylpyrazol-3-yl)pyrrolo[2,3-b]pyridin-5-yl}phenyl)(4-methylpiperazin-1-yl)ketone with a yield of 63.7%. ¹H NMR (600 MHz, Chloroform-d) δ 8.72 (d, *J =* 2.0 Hz, 1H), 8.21 - 8.14 (m, 2H), 8.00 (d, *J* = 2.2 Hz, 1H), 7.88 (t, *J =* 1.4 Hz, 1H), 7.63 - 7.57 (m, 3H), 7.51 (d, *J =* 7.8 Hz, 2H), 7.34 (d, *J =* 8.1 Hz, 2H), 6.45 (q, *J =* 1.7 Hz, 1H), 3.95 (t, *J =* 1.5 Hz, 3H), 3.93 - 3.47 (m, 4H), 2.57 (s, 3H), 2.41 (s, 4H), 2.40 (s, 3H). ¹³C NMR (151 MHz, CDCl₃) δ 169.78, 146.43, 145.82, 144.89, 139.56, 139.05, 134.94, 133.81, 132.32, 129.89, 128.35, 128.03, 127.56, 127.13, 125.17, 121.96, 109.20, 107.04, 54.66, 45.75, 41.76, 37.62, 24.88, 21.73.

### 2) Synthesis of (4-methylpiperazin-1-yl){4-[3-(2-methylpyrazol-3-yl)-1H-pyrrolo[2,3-b]pyridin-5-yl]phenyl } ketone

Into a 10 mL single-necked flask, (4-{1-[(4-methylphenyl)dioxo-λ⁶-thio]-3-(2-methylpyrazol-3-yl)pyrrolo[2,3-b]pyridin-5-yl}phenyl)(4-methylpiperazin-1-yl)ketone (1.0 eq., 0.15 mmol, 82 mg) and potassium carbonate (3.0 eq., 0.44 mmol, 61 mg) were added, and 5 mL of methanol was added. The mixture was allowed to react at 80°C for 4 h. After concentration under reduced pressure, it was purified by column chromatography (DCM:MeOH=10:1) to obtain 27 mg of (4-methylpiperazin-1-yl){4-[3-(2-methylpyrazol-3-yl)-1H-pyrrolo[2,3-b]pyridin-5-yl]phenyl}ketone with a yield of 46.1%. ¹H NMR (600 MHz, Chloroform-d) δ 8.66 (s, 1H), 8.14 (s, 1H), 7.71 - 7.65 (m, 2H), 7.62 (d, *J* = 2.4 Hz, 1H), 7.54 (d, *J =* 7.4 Hz, 3H), 6.46 (d, *J* = 2.5 Hz, 1H), 3.96 (d, *J =* 2.6 Hz, 3H), 3.92 - 3.55 (m, 4H), 2.50 (d, *J =* 68.0 Hz, 4H), 2.38 (d, *J =* 2.5 Hz, 3H).¹³C NMR (151 MHz, CDCl₃) δ 170.03, 148.16, 143.16, 140.57, 138.89, 135.99, 134.55, 129.85, 127.95, 127.48, 126.72, 124.85, 119.36, 106.42, 105.61, 58.44, 55.22, 54.71, 45.92, 37.43.

### Example 15: Synthesis of compound 15, 5-{4-[(4-methylpiperazin-1-yl)methyl]phenyl}-3-(2-methylpyrazol-3-yl)-1H-pyrrolo[2,3-b]pyridine

### 1) Synthesis of 1-[(4-methylphenyl)dioxo-λ⁶-thio]-5-{4-[(4-methylpiperazin-1-yl)methyl]phenyl}-3-(2-methylpyrazol-3-yl)pyrrolo[2,3-b]pyridine

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-(4-methyl-1-piperazinemethyl)phenylboronic acid pinacol ester (1.5 eq., 0.35 mmol, 110 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 77 mg of 1-[(4-methylphenyl)dioxo-λ⁶-thio]-5-{4-[(4-methylpiperazin-1-yl)methyl]phenyl}-3-(2-methylpyrazol-3-yl)pyrrolo[2,3-b]pyridine with a yield of 61.5%. ¹H NMR (600 MHz, Chloroform-d) δ 8.85 (d, *J =* 2.0 Hz, 1H), 8.80 (d, *J =* 2.0 Hz, 1H), 7.74 (d, *J =* 8.2 Hz, 2H), 7.69 (d, *J =* 1.9 Hz, 1H), 7.61 (d, *J =* 8.0 Hz, 2H), 7.53 - 7.49 (m, 1H), 7.43 (d, *J =* 7.8 Hz, 2H), 7.26 (d, *J =* 8.2 Hz, 2H),6.49 (d, *J =* 1.8 Hz, 1H), 3.98 (s, 3H), 3.68 (s, 2H), 2.69 (s, 8H), 2.62 (s, 3H), 2.52 (s, 3H).

### 2) Synthesis of 5-{4-[(4-methylpiperazin-1-yl)methyl]phenyl}-3-(2-methylpyrazol-3-yl)-1H-pyrrolo[2,3-b]pyridine

Into a 10 mL single-necked flask, 1-[(4-methylphenyl)dioxo-λ⁶-thio]-5-{4-[(4-methylpiperazin-1-yl)methyl]phenyl}-3-(2-methylpyrazol-3-yl)pyrrolo[2,3-b]pyridine (1.0 eq., 0.14 mmol, 77 mg) and potassium carbonate (3.0 eq., 0.43 mmol, 59 mg) were added, and 5 mL of methanol was added. The mixture was allowed to react at 80°C for 4 h. After concentration under reduced pressure, it was purified by column chromatography (DCM:MeOH=10:1) to obtain 25 mg of 5-{4-[(4-methylpiperazin-1-yl)methyl]phenyl}-3-(2-methylpyrazol-3-yl)-1*H-*pyrrolo[2,3-b]pyridine with a yield of 45.3%.

¹H NMR (600 MHz, Chloroform-*d*) δ 8.65 (d, *J =* 2.0 Hz, 1H), 8.12 (d, *J =* 2.0 Hz, 1H), 7.61 (d, *J =* 1.9 Hz, 1H), 7.58 (d, *J =* 8.0 Hz, 2H), 7.53 - 7.49 (m, 1H), 7.43 (d, *J =* 7.8 Hz, 2H), 6.46 (d, *J =* 1.8 Hz, 1H), 3.95 (s, 3H), 3.62 (s, 2H), 2.69 (s, 8H), 2.48 (s, 3H).¹³C NMR (151 MHz, CDCl₃) δ 147.90, 143.38, 138.86, 136.12, 130.52, 129.89, 128.89, 127.39, 126.52, 125.98, 124.53, 119.22, 106.38, 105.57, 62.23, 54.69, 51.86, 45.21, 37.44.

### Example 16: Synthesis of compound 16, 3-(2-methylpyrazol-3-yl)-5-[4-(1,4-oxazacyclohexan-4-yl)phenyl]-1H-pyrrolo[2,3-b]pyridine

### 1) Synthesis of 1-[(4-methylphenyl)dioxo-λ⁶-thio]-3-(2-methylpyrazol-3-yl)-5-[4-(1,4-oxazacyclohexan-4-yl)phenyl]pyrrolo[2,3-b]pyridine

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-morpholinylphenylboronic acid pinacol ester (1.5 eq., 0.35 mmol, 101 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 74 mg of 1-[(4-methylphenyl)dioxo-λ⁶-thio]-3-(2-methylpyrazol-3-yl)-5-[4-(1,4-oxazacyclohexan-4-yl)phenyl]pyrrolo[2,3-b]pyridine with a yield of 62.2%. ¹H NMR (600 MHz, Chloroform-d) δ 8.70 (d, *J =* 2.4 Hz, 1H), 8.16 (d, *J =* 8.0 Hz, 2H), 7.94 (d, *J* = 2.2 Hz, 1H), 7.87 - 7.81 (m, 1H), 7.60 (d, *J =* 2.3 Hz, 1H), 7.50 (d, *J =* 8.1 Hz, 2H), 7.33 (d, *J* = 8.1 Hz, 2H), 7.13 (s, 2H), 6.44 (d, *J =* 2.4 Hz, 1H), 4.01 - 3.89 (m, 7H), 3.26 (s, 4H), 2.40 (s, 3H).¹³C NMR (151 MHz, CDCl₃) δ 145.95, 145.67, 144.72, 138.99, 135.06, 134.06, 129.84, 128.30, 126.39, 124.91, 121.92, 109.18, 107.01, 66.40, 58.49, 37.62, 21.72.

### 2) Synthesis of 3-(2-methylpyrazol-3-yl)-5-[4-(1,4-oxazacyclohexan-4-yl)phenyl]-1H-pyrrolo[2,3-b]pyridine

Into a 10 mL single-necked flask, 1-[(4-methylphenyl)dioxo-λ⁶-thio]-3-(2-methylpyrazol-3-yl)-5-[4-(1,4-oxazacyclohexan-4-yl)phenyl]pyrrolo[2,3-b]pyridine (1.0 eq., 0.14 mmol, 74 mg) and potassium carbonate (3.0 eq., 0.43 mmol, 59 mg) were added, and 5 mL of methanol was added. The mixture was allowed to react at 80°C for 4 h. After concentration under reduced pressure, it was purified by column chromatography (DCM:MeOH=10:1) to obtain 24 mg of 3-(2-methylpyrazol-3-yl)-5-[4-(1,4-oxazacyclohexan-4-yl)phenyl]-1*H*-pyrrolo[2,3-b]pyridine with a yield of 46.8%. ¹H NMR (600 MHz, Chloroform-d) δ 8.62 (s, 1H), 8.12 (d, *J =* 2.3 Hz, 1H), 7.61 (s, 1H), 7.60 - 7.53 (m, 2H), 7.51 (d, *J =* 2.4 Hz, 1H), 7.08 - 6.96 (m, 2H), 6.45 (s, 1H), 3.95 (d, *J* = 2.1 Hz, 3H), 3.90 (dt, *J =* 5.7, 2.8 Hz, 4H), 3.23 (p, *J =* 2.6 Hz, 4H). ¹³C NMR (151 MHz, CDCl₃) δ 150.72, 146.60, 141.89, 138.88, 135.95, 130.66, 129.99, 128.14, 126.76, 124.77, 119.86, 116.05, 106.46, 105.65, 66.83, 49.14, 37.45.

### Example 17: Synthesis of compound 17, 3-(2-methylpyrazol-3-yl)-5-[4-(piperazin-1-yl)phenyl]-1H-pyrrolo[2,3-b]pyridine

### 1) Synthesis of 2-methylprop-2-yl 4-(4-{1-[(4-methylphenyl)dioxo-λ⁶-thio]-3-(2-methylpyrazol-3-yl)pyrrolo[2,3-b]pyridin-5-yl}phenyl)piperazine-1-formate

Into a 50 mL two-necked flask, 5-bromo-1-[(4-methylphenyl)dioxo-λ⁶-thio]-3-(2-methylpyrazol-3-yl)pyrrolo[2,3-b]pyridine (1.0 eq., 0.26 mmol, 100 mg), 4-[4-(N-BOC)piperazin-1-yl]phenylboronic acid pinacol ester (1.5 eq., 0.35 mmol, 135 mg), potassium carbonate (3.0 eq., 0.70 mmol, 96 mg), [1,1'-bis(diphenylphosphino)ferrocene]dichloropalladium (II) (0.05 eq., 0.01 mmol, 8 mg) were added, then 4 mL of 1,4-dioxane and 1 mL of water were added, the atmosphere was replaced with argon three times, and the mixture was heated under reflux to react overnight. After the reaction was cooled, water and DCM were added for extraction. The organic phase was taken, washed with saturated sodium chloride solution, and then dried over anhydrous sodium sulfate. After concentration under reduced pressure, the system was purified by column chromatography (DCM:MeOH=20:1) to obtain 98 mg of 2-methylprop-2-yl 4-(4-{1-[(4-methylphenyl)dioxo-λ⁶-thio]-3-(2-methylpyrazol-3-yl)pyrrolo[2,3-b]pyridin-5-yl}phenyl)piperazine-1-formate with a yield of 69.1%.

### 2) Synthesis of 2-methylprop-2-yl 4-{4-[3-(2-methylpyrazol-3-yl)-1H-pyrrolo[2,3-b]pyridin-5-yl]phenyl}piperazine-1-formate

Into a 10 mL single-necked flask, 2-methylprop-2-yl 4-(4-{1-[(4-methylphenyl)dioxo-λ⁶-thio]-3-(2-methylpyrazol-3-yl)pyrrolo[2,3-b]pyridin-5-yl}phenyl)piperazine-1-formate (1.0 eq., 0.16 mmol, 98 mg) and potassium carbonate (3.0 eq., 0.48 mmol, 66 mg) were added, and 5 mL of methanol was added. The mixture was allowed to react at 80°C for 4 h. After concentration under reduced pressure, it was purified by column chromatography (DCM:MeOH=10:1) to obtain 47 mg of 2-methylprop-2-yl 4-{4-[3-(2-methylpyrazol-3-yl)-1*H*-pyrrolo[2,3-b]pyridin-5-yl]phenyl} piperazine-1-formate with a yield of 63.6%. ¹H NMR (600 MHz, Chloroform-d) δ 8.55 (s, 1H), 8.30 (s, 1H), 7.63 (s, 1H), 7.58 (s, 1H), 7.52 (d, *J* = 8.2 Hz, 2H), 7.08 (d, *J =* 7.9 Hz, 2H), 6.47 (d, *J =* 3.1 Hz, 1H), 3.94 (t, *J =* 1.7 Hz, 3H), 3.64 (s, 4H), 3.24 (d, *J =* 6.0 Hz, 4H), 1.50 (t, *J =* 1.7 Hz, 9H).

### 3) Synthesis of 3-(2-methylpyrazol-3-yl)-5-[4-(piperazin-1-yl)phenyl]-1H-pyrrolo[2,3-b]pyridine

Into a 10 mL single-necked flask, 47 mg of 2-methylprop-2-yl 4-{4-[3-(2-methylpyrazol-3-yl)-1*H*-pyrrolo[2,3-b]pyridin-5-yl]phenyl } piperazine-1-formate was added, DCM containing 10% trifluoroacetic acid was added, and the mixture was stirred at room temperature overnight. After the reaction was completed, the product was spin-dried to obtain 34 mg of the final product, 3-(2-methylpyrazol-3-yl)-5-[4-(piperazin-1-yl)phenyl]-1*H*-pyrrolo[2,3-b]pyridine with a yield of 92.3%. ¹H NMR (600 MHz, DMSO-*d*₆) δ 8.57 (s, 1H), 8.09 (s, 1H), 7.85 (s, 1H), 7.64 (s, 2H), 7.51 (s, 1H), 7.07 (s, 2H), 6.59 (s, 1H), 3.92 (s, 3H), 3.16 (d, *J =* 67.6 Hz, 8H). ¹³C NMR (151 MHz, DMSO) δ 147.43, 142.05, 137.88, 135.89, 128.81, 127.55, 125.81, 124.17, 118.03, 116.08, 105.11, 103.48, 46.65, 43.48, 37.31.

### Example 18: Synthesis of compound 18, 5-(4-{[2-(diethylamino)ethyl]oxy}phenyl)-3-(2-methylpyrazol-3-yl)-1H-pyrrolo[2,3-b]pyridine

### 1) Synthesis of 4-{1-[(4-methylphenyl)dioxo-λ⁶-thio]-3-(2-methylpyrazol-3-yl)pyrrolo[2,3-b]pyridin-5-yl}phenol

Into a 100 mL three-necked flask, 5-bromo-1-[(4-methylphenyl)dioxo-λ⁶-thio]-3-(2-methylpyrazol-3-yl)pyrrolo[2,3-b]pyridine (1.0 eq., 1.62 mmol, 700 mg), 4-hydroxyphenylboronic acid pinacol ester (1.5 eq., 2.43 mmol, 536 mg), [1,1'-bis(diphenylphosphino) ferrocene]dichloropalladium (II) (0.05 eq., 0.081 mmol, 59 mg), potassium carbonate (3.0 eq., 4.86 mmol, 672 mg) were added, then 20 mL of 1,4-dioxane and 5 mL of water were added, the atmosphere was replaced with argon three times, and the mixture was heated under reflux at 100°C to react overnight. After the reaction was cooled, water and DCM were added for extraction. The organic phase was taken, washed with water three times, washed with saturated sodium chloride solution three times, and then dried over anhydrous sodium sulfate. After concentration under reduced pressure, the system was purified by column chromatography (DCM:MeOH=30:1) to obtain 418 mg of 4-{1-[(4-methylphenyl)dioxo-λ⁶-thio]-3-(2-methylpyrazol-3-yl)pyrrolo[2,3-b]pyridin-5-yl}phenol with a yield of 58.0%.

### 2) Synthesis of 2-[(4-{1-[(4-methylphenyl)dioxo-λ⁶-thio]-3-(2-methylpyrazol-3-yl)pyrrolo [2,3-b]pyridin-5-yl}phenyl)oxy]ethan-1-ol

Into a 50 ml flask, 4-{1-[(4-methylphenyl)dioxo-λ⁶-thio]-3-(2-methylpyrazol-3-yl)pyrrolo[2,3-b]pyridin-5-yl}phenol (1.0 eq., 0.90 mmol, 400 mg), 2-bromoethanol (1.2 eq., 1.08 mmol, 135 mg), potassium carbonate (3.0 eq., 2.70 mmol, 373 mg) and 10 ml of acetone were added and heated at 55°C overnight. After the reaction was cooled, the solvent was concentrated under reduced pressure for spin-drying, water and DCM were added for extraction. The organic phase was taken, washed with water three times, washed with saturated sodium chloride solution three times, and then dried over anhydrous sodium sulfate. After concentration under reduced pressure, the system was purified by column chromatography (DCM:MeOH=30:1) to obtain 237 mg of 2-[(4-{1-[(4-methylphenyl)dioxo-λ⁶-thio]-3-(2-methylpyrazol-3-yl)pyrrolo[2,3-b]pyridin-5-yl}phenyl)oxy]ethan-1-ol with a yield of 54.0%

### 3) Synthesis of 4-methylbenzenesulfonic acid-2-[(4-{1-[(4-methylphenyl)dioxo-λ⁶-thio]-3-(2-methylpyrazol-3-yl)pyrrolo[2,3-b]pyridin-5-yl}phenyl)oxy]ethyl ester

Into a 50 ml flask, 2-[(4-{1-[(4-methylphenyl)dioxo-λ⁶-thio]-3-(2-methylpyrazol-3-yl)pyrrolo[2,3-b]pyridin-5-yl}phenyl)oxy]ethan-1-ol (1.0 eq., 0.41 mmol, 200 mg), p-toluenesulfonyl chloride (1.2 eq., 0.49 mmol, 94 mg), triethylamine (5.0 eq., 2.05 mmol, 207 mg), 10 mL of DCM were added, and the mixture was stirred to react at room temperature for 8 h. After the reaction was completed, the system was washed with water. The organic phase was taken, washed with saturated sodium chloride solution three times, and then dried over anhydrous sodium sulfate. After concentration under reduced pressure, the system was purified by column chromatography (DCM:MeOH=30:1) to obtain 218 mg of 4-methylbenzenesulfonic acid-2-[(4-{1-[(4-methylphenyl)dioxo-λ⁶-thio]-3-(2-methylpyrazol-3-yl)pyrrolo[2,3-b]pyridin-5-yl}phenyl)oxy]ethyl ester with a yield of 83.0%.

### 4) Synthesis of 5-(4-{[2-(diethylamino)ethyl]oxy}phenyl)-1-[(4-methylphenyl)dioxo-λ⁶-thio]-3-(2-methylpyrazol-3-yl)pyrrolo[2,3-b]pyridine

Into a 25 mL flask, 4-methylbenzenesulfonic acid-2-[(4-{1-[(4-methylphenyl)dioxo-λ⁶-thio]-3-(2-methylpyrazol-3-yl)pyrrolo[2,3-b]pyridin-5-yl}phenyl)oxy]ethyl ester (1.0 eq., 0.23 mmol, 150 mg), diethylamine (2.2 eq., 0.51 mmol, 37.55 mg), potassium carbonate (1.1 eq., 0.26 mmol, 36 mg) were added, then 5 mL of acetonitrile was added, and the mixture was then heated under reflux to react overnight. After the reaction was cooled, it was concentrated under reduced pressure and spin-dried without any other post-treatment to obtain crude compound 5-(4-{[2-(diethylamino)ethyl]oxy}phenyl)-1-[(4-methylphenyl)dioxo-λ⁶-thio]-3-(2-methylpyrazol-3-yl)pyrrolo[2,3-b]pyridine, which was directly subjected to the next step of reaction.

### 5) Synthesis of 5-(4-{[2-(diethylamino)ethyl]oxy } phenyl)-3-(2-methylpyrazol-3-yl)-1H-pyrrolo[2,3-b]pyridine

Into a 25 mL single-necked flask, 5-(4-{[2-(diethylamino)ethyl]oxy}phenyl)-1-[(4-methylphenyl)dioxo-λ⁶-thio]-3-(2-methylpyrazol-3-yl)pyrrolo[2,3-b]pyridine and potassium carbonate (2.0 eq., 0.46 mmol, 65 mg) were added, and 5 mL of methanol was added. The mixture was allowed to react at 80°C for 16 h. After the reaction was cooled, water and DCM were added for extraction. The organic phase was taken, washed with saturated sodium chloride solution, and then dried over anhydrous sodium sulfate. After concentration under reduced pressure, it was purified by column chromatography (DCM:MeOH=30:1) to obtain 40 mg of 5-(4-{[2-(diethylamino)ethyl]oxy}phenyl)-3-(2-methylpyrazol-3-yl)-1*H*-pyrrolo[2,3-b]pyridine. The total yield of the two-step reaction was 44.0%. ¹H NMR (600 MHz, Methanol-*d*₄) δ 8.54 (d, J = 2.0 Hz, 1H), 8.08 (d, J = 2.1 Hz, 1H), 7.77 (s, 1H), 7.58 (d, J = 1.9 Hz, 1H), 7.49 - 7.44 (m, 2H), 6.94 - 6.86 (m, 2H), 6.53 (d, J = 2.0 Hz, 1H), 4.52 (t, J = 6.7 Hz, 2H), 3.95 (d, J = 2.1 Hz, 3H), 3.05 (t, J = 6.6 Hz, 2H), 2.72 (q, J = 7.1 Hz, 4H), 1.05 (t, J = 7.2 Hz, 6H). ¹³C NMR (151 MHz, Methanol-*d*₄) δ 158.36, 147.65, 143.42, 139.66, 138.25, 132.07, 131.49, 130.51, 129.48, 126.84, 121.01, 116.94, 106.98, 104.45, 53.37, 49.43, 49.28, 49.14, 49.00, 48.86, 48.72, 48.57, 48.40, 43.71, 37.57, 11.76.

### Example 19: Synthesis of compound 19, 3-(3,5-dimethylisoxazol-4-yl)-5-[4-(4-methylpiperazin-1-yl)phenyl]-1H-pyrrolo[2,3-b]pyridine

### 1) Synthesis of compound 3-(3,5-dimethylisoxazol-4-yl)-1-[(4-methylphenyl)dioxo-λ6-thio]-5-[4-(4-methylpiperazin-1-yl)phenyl]pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 3,5-dimethylisoxazole-4-boronic acid pinacol ester (1.5 eq., 0.39 mmol, 88 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to obtain 90 mg of the compound **3-(3,5-dimethylisoxazol-4-yl)-1-[(4-methylphenyl)dioxo-**λ**6-thio]-5-[4-(4-methylpiperazin-1-yl)phenyl]pyrrolo[2,3-b]pyridine** with a yield of 63.6%. ¹H NMR (600 MHz, Chloroform-*d*) δ 8.68 (d, *J =* 2.5 Hz, 1H), 8.19 - 8.13 (m, 2H), 7.71 (d, *J =* 2.5 Hz, 1H), 7.67 (d, *J =* 2.0 Hz, 1H), 7.45 - 7.41 (m, 2H), 7.33 (d, *J =* 8.0 Hz, 2H), 7.03 - 6.98 (m, 2H), 3.30 (t, *J =* 4.9 Hz, 4H), 2.63 (s, 4H), 2.40 (d, *J* = 5.1 Hz, 6H), 2.37 (d, *J =* 2.1 Hz, 3H), 2.22 (d, *J =* 2.0 Hz, 3H).¹³C NMR (151 MHz, CDCl₃) δ 166.89, 159.44, 150.84, 146.03, 145.49, 144.43, 135.22, 132.78, 129.79, 128.78, 128.23, 128.01, 125.78, 125.22, 122.37, 116.24, 108.30, 107.63, 54.92, 48.51, 46.01, 21.71, 11.79, 10.78.

### 2) Synthesis of compound 3-(3,5-dimethylisoxazol-4-yl)-5-[4-(4-methylpiperazin-1-yl)phenyl]-1H-pyrrolo[2,3-b]pyridine

Into a 10 mL single-necked flask, **3-(3,5-dimethylisoxazol-4-yl)-1-[(4-methylphenyl)dioxo-**λ**6-thio]-5-[4-(4-methylpiperazin-1-yl)phenyl]pyrrolo[2,3-b]pyridine** (1.0 eq., 0.17 mmol, 90 mg) and potassium carbonate (3.0 eq., 0.50 mmol, 69 mg) were added, and 5 mL of methanol was added. The mixture was allowed to react at 80°C for 4 h. After concentration under reduced pressure, it was purified by column chromatography (DCM:MeOH=10:1) to obtain 42 mg of compound **3-(3,5-dimethylisoxazol-4-yl)-5-[4-(4-methylpiperazin-1-yl)phenyl]-1H-pyrrolo [2,3-b]pyridine** with a yield of 64.6%. ¹H NMR (600 MHz, DMSO-d₆) δ 8.53 (d, *J =* 2.6 Hz, 1H), 7.92 (d, *J =* 2.6 Hz, 1H), 7.64 (d, *J =* 2.9 Hz, 1H), 7.59 (d, *J =* 8.5 Hz, 2H), 7.04 (d, *J =* 8.6 Hz, 2H), 3.32 (s, 3H), 3.26 (s, 4H), 2.72 (s, 4H), 2.41 (s, 3H), 2.20 (d, *J =* 2.1 Hz, 3H). ¹³C NMR (151 MHz, DMSO) δ 165.12, 159.12, 149.54, 147.55, 141.58, 140.97, 128.41, 127.40, 127.24, 126.15, 123.77, 118.54, 115.80, 109.23, 53.76, 47.15, 11.42, 10.36. ESI-MS *m*/*z* 388.4 [M+H]⁺. ESI-HRMS (m/z): Calculated for [C₂₃H₂₅N₅O]⁺: 388.4274, found 388.4276.

### Example 20: Synthesis of compound 20, 3-(1H-pyrazol-3-yl)-5-(4-[[2-(1,4-oxazacyclohexan-4-yl)ethyl]oxy]phenyl)-1H-pyrrolo[2,3-b]pyridine

### 1) Synthesis of compound 3-(1H-pyrazol-3-yl)-1-[(4-methylphenyl)dioxo-λ6-thio]-5-(4-[[2-(1,4-oxazacyclohexan-4-yl)ethyl]oxy]phenyl)pyrrolo[2,3-b]pyridine

Into a 50 mL two-necked flask, 4-(2-(4-(3-iodo-1-p-toluenesulfonyl-1H-pyrrolo[2,3-b]pyridin-5-yl)phenoxy)ethyl)morpholine (1.0 eq., 0.26 mmol, 150 mg), 1H-pyrazole-3-boronic acid pinacol ester (1.5 eq., 0.39 mmol, 76 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to give 83 mg of **3-(1H-pyrazol-3-yl)-1-[(4-methylphenyl)dioxo-λ6-thio]-5-(4-[[2-(1,4-oxazacyclohexan-4-yl)ethyl]oxy]phenyl)pyrrolo[2,3-b]pyridine** with a yield of 62.4%. ¹H NMR (600 MHz, Chloroform-*d*) δ 8.64 (d, *J* = 2.4 Hz, 1H), 8.58 (s, 1H), 8.11 (dd, *J =* 8.5, 2.0 Hz, 2H), 8.03 (s, 1H), 7.66 (s, 1H), 7.53 - 7.47 (m, 2H), 7.28 (s, 2H), 7.00 - 6.95 (m, 2H), 6.64 (s, 1H), 4.20 (d, *J =* 5.9 Hz, 2H), 3.79 (d, *J =* 5.2 Hz, 4H), 2.89 (s, 2H), 2.67 (s, 4H), 2.36 (d, *J* = 1.9 Hz, 3H).¹³C NMR (151 MHz, CDCl₃) δ 158.36, 146.60, 145.25, 144.27, 143.57, 135.34, 132.61, 131.24, 129.70, 128.56, 128.49, 128.05, 124.82, 123.46, 121.22, 115.10, 113.17, 103.13, 66.66, 65.62, 57.58, 54.01, 21.64. ESI-MS *m*/*z* 544.4 [M+H]⁺.

### 2) Synthesis of compound 3-(1H-pyrazol-3-yl)-5-(4-[[2-(1,4-oxazacyclohexan-4-yl)ethyl]oxy]phenyl)-1H-pyrrolo[2,3-b]pyridine

Into a 10 mL single-necked flask, **3-(1H-pyrazol-3-yl)-1-[(4-methylphenyl)dioxo-**λ**6-thio]-5-(4-[[2-(1,4-oxazacyclohexan-4-yl)ethyl]oxy]phenyl)pyrrolo[2,3-b]pyridine** (1.0 eq., 0.16 mmol, 83 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 24 mg of **3-(1H-pyrazol-3-yl)-5-(4-[[2-(1,4-oxazacyclohexan-4-yl)ethyl]oxy]phenyl)-1H-pyrrolo[2,3-b]pyridine** with a yield of 59.4%. ¹H NMR (600 MHz, Methanol-*d*₄) δ 7.64 (s, 1H), 7.56 (d, *J =* 2.1 Hz, 1H), 6.87 (s, 1H), 6.79 (d, *J* = 2.2 Hz, 1H), 6.77 - 6.70 (m, 2H), 6.18 (d, *J =* 8.6 Hz, 2H), 5.77 (d, *J =* 2.2 Hz, 1H), 3.31 (t, *J =* 5.4 Hz, 2H), 2.84 (t, *J =* 4.6 Hz, 4H), 1.96 (t, *J =* 5.4 Hz, 2H), 1.75 (t, *J =* 4.6 Hz, 4H).¹³C NMR (151 MHz, DMSO) δ 157.65, 147.87, 141.59, 131.35, 128.30, 127.91, 125.99, 123.92, 117.02, 114.98, 101.30, 66.09, 65.32, 56.93, 53.55. ESI-MS *m*/*z* 390.2 [M+H]⁺. ESI-HRMS (m/z): Calculated for [C₂₂H₂₃N₅O₂]⁺: 390.1852, found 390.1853.

### Example 21: Synthesis of compound 21, 5-(4-[[2-(hexahydropyridin-1-yl)ethyl]oxy]phenyl)-3-(1H-pyrazol-3-yl)-1H-pyrrolo[2,3-b]pyridine

### 1) Synthesis of compound 5-(4-[[2-(hexahydropyridin-1-yl)ethyl]oxy]phenyl)3-(1H-pyrazol-3-yl)-1-[(4-methylphenyl)dioxo-λ6-thio]pyrrolo[2,3-b]pyridine

Into a 50 mL two-necked flask, 3-iodo-5-(4-(2-(piperidin-1-yl)ethoxy)phenyl)-1-p-toluenesulfonyl-1H-pyrrolo[2,3-b]pyridine (1.0 eq., 0.26 mmol, 150 mg), 1H-pyrazole-3-boronic acid pinacol ester (1.5 eq., 0.39 mmol, 76 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to give 65 mg of **5-(4-[[2-(hexahydropyridin-1-yl)ethyl]oxy]phenyl)3-(1H-pyrazol-3-yl)-1-[(4-methylphenyl)dioxo-**λ**6-thio]pyrrolo[2,3-b]pyridine** with a yield of 42.3%. ¹H NMR (600 MHz, Chloroform-d) δ 8.62 (d, *J* = 2.1 Hz, 1H), 8.57 (s, 1H), 8.10 (d, *J =* 8.1 Hz, 2H), 8.02 (s, 1H), 7.66 (d, *J* = 2.4 Hz, 1H), 7.47 (d, *J =* 8.3 Hz, 2H), 7.27 (s, 1H), 6.94 (d, *J =* 8.4 Hz, 2H), 6.63 (d, *J* = 2.3 Hz, 1H), 4.23 (t, *J =* 5.6 Hz, 2H), 3.00 (t, *J* = 5.5 Hz, 2H), 2.75 (s, 4H), 2.36 (s, 3H), 1.72 (p, *J =* 5.6 Hz, 4H), 1.52 (s, 2H). ¹³C NMR (151 MHz, CDCl₃) δ 158.13, 146.59, 145.26, 144.24, 135.31, 132.57, 131.28, 129.70, 128.55, 128.03, 123.39, 121.24, 115.05, 103.05, 65.06, 57.37, 54.72, 25.01, 23.51, 21.64. ESI-MS *m*/*z* 542.2 [M+H]⁺.

### 2) Synthesis of 5-(4-[[2-(hexahydropyridin-1-yl)ethyl]oxy]phenyl)-3-(1H-pyrazol-3-yl)-1H-pyrrolo[2,3-b]pyridine

Into a 10 mL single-necked flask, **5-(4-[[2-(hexahydropyridin-1-yl)ethyl]oxy]phenyl)3-(1H-pyrazol-3-yl)-1-[(4-methylphenyl)dioxo-λ6-thio]pyrrolo[2,3-b]pyridine** (1.0 eq., 0.16 mmol, 83 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 32 mg of **5-(4-[[2-(hexahydropyridin-1-yl)ethyl]oxy]phenyl)-3-(1H-pyrazol-3-yl)-1H-pyrrolo[2,3-b]pyridine** with a yield of 53.4%. ¹H NMR (600 MHz, DMSO-*d*₆) δ 8.56 (s, 1H), 8.51 (s, 1H), 7.88 (s, 1H), 7.70 (s, 1H), 7.68 - 7.57 (m, 2H), 7.07 (d, *J =* 8.1 Hz, 2H), 6.68 (s, 1H), 4.12 (t, *J =* 6.1 Hz, 2H), 2.69 (d, *J =* 7.8 Hz, 2H), 2.46 (s, 4H), 1.51 (p, *J* = 5.6 Hz, 4H), 1.39 (q, *J =* 6.1, 5.7 Hz, 2H).¹³C NMR (151 MHz, DMSO) δ 157.69, 147.86, 141.56, 131.29, 128.30, 127.89, 123.91, 114.97, 101.30, 65.57, 57.26, 54.31, 25.47, 23.82. ESI-MS *m*/*z* 388.2 [M+H]⁺. ESI-HRMS (m/z): Calculated for [C₂₃H₂₅N₅O]⁺: 388.2259, found 388.2259.

### Example 22: Synthesis of compound 22, 5-[4-(4-methylpiperazin-1-yl)phenyl]-3-(pyridin-3-yl)-1H-pyrrolo[2,3-b]pyridine:

### 1) Synthesis of compound 1-[(4-methylphenyl)dioxo-16-thio]-5-[4-(4-methylpiperazin-1-yl)phenyl]-3-(pyridin-3-yl)pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 3-pyridinboronic acid pinacol ester (1.5 eq., 0.39 mmol, 80 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to obtain 83 mg of the compound 1-[(4-methylphenyl)dioxo-λ6-thio]-5-[4-(4-methylpiperazin-1-yl)phenyl]-3-(pyridin-3-yl)pyrrolo[2,3-b]pyridine with a yield of 60.9%. ¹H NMR (600 MHz, Chloroform-*d*) δ 8.91 (s, 1H), 8.69 (d, *J* = 2.4 Hz, 1H), 8.66 - 8.59 (m, 1H), 8.16 (s, 1H), 8.14 (t, *J =* 2.5 Hz, 2H), 7.93 (d, *J =* 2.3 Hz, 1H), 7.91 (d, *J =* 8.0 Hz, 1H), 7.52 - 7.45 (m, 2H), 7.41 (dt, *J =* 7.5, 3.3 Hz, 1H), 7.31 (d, *J =* 8.1 Hz, 2H), 7.01 (dd, *J =* 8.8, 2.5 Hz, 2H), 3.33 (s, 4H), 2.69 (s, 4H), 2.43 (s, 3H), 2.39 (d, *J =* 2.3 Hz, 3H).¹³C NMR (151 MHz, CDCl₃) δ 150.69, 148.83, 148.45, 146.43, 145.48, 144.47, 135.21, 134.67, 132.94, 129.78, 129.14, 128.91, 128.22, 128.14, 126.00, 123.91, 123.72, 121.16, 116.89, 116.37, 54.80, 48.40, 45.82, 21.69. ESI-MS *m*/*z* 524.4 [M+H]⁺.

### 2) Synthesis of compound 5-[4-(4-methylpiperazin-1-yl)phenyl]-3-(pyridin-3-yl)-1H-pyrrolo[2,3-b]pyridine

Into a 10 mL single-necked flask, 1-[(4-methylphenyl)dioxo-16-thio]-5-[4-(4-methylpiperazin-1-yl)phenyl]-3-(pyridin-3-yl)pyrrolo[2,3-b]pyridine (1.0 eq., 0.16 mmol, 83 mg) and potassium carbonate (3.0 eq., 0.48 mmol, 66 mg) were added, and 5 mL of methanol was added. The mixture was allowed to react at 80°C for 4 h. After concentration under reduced pressure, it was purified by column chromatography (DCM:MeOH=10:1) to obtain 38 mg of 5-[4-(4-methylpiperazin-1-yl)phenyl]-3-(pyridin-3-yl)-1*H*-pyrrolo[2,3-*b*]pyridine with a yield of 65.3%. ¹H NMR (600 MHz, DMSO-*d*₆) δ 9.02 (s, 1H), 8.54 (s, 1H), 8.46 (d, *J =* 4.7 Hz, 1H), 8.37 (s, 1H), 8.20 (d, *J =* 8.0 Hz, 1H), 8.03 (s, 1H), 7.64 (d, *J =* 8.2 Hz, 2H), 7.46 (dd, *J =* 7.9, 4.8 Hz, 1H), 7.05 (d, *J =* 8.3 Hz, 2H), 3.19 (t, *J* = 4.8 Hz, 4H), 2.48 (d, *J* = 6.2 Hz, 4H), 2.24 (s, 3H).¹³C NMR (151 MHz, CDCl₃) δ 150.41, 147.77, 146.97, 142.39, 134.48, 131.28, 130.46, 130.40, 130.22, 128.15, 125.98, 125.69, 124.04, 123.66, 118.48, 116.61, 112.66, 54.86, 48.71, 45.84. ESI-MS *m*/*z* 370.3 [M+H]⁺. ESI-HRMS (m/z): Calculated for [C₂₃H₂₃N₅]⁺: 370.3245, found 370.3242.

### Example 23: Synthesis of compound 23, 5-[4-(4-methylpiperazin-1-yl)phenyl]-3-(pyrimidin-5-yl)-1H-pyrrolo[2,3-b]pyridine

### 1) Synthesis of compound 1-[(4-methylphenyl)dioxo-λ6-thio]-5-[4-(4-methylpiperazin-1-yl)phenyl]-3-(pyrimidin-5-yl)pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), pyrimidin-5-boronic acid pinacol ester (1.5 eq., 0.39 mmol, 81 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to obtain 84 mg of the compound 1-[(4-methylphenyl)dioxo-λ⁶-thio]-5-[4-(4-methylpiperazin-1-yl)phenyl]-3-(pyrimidin-5-yl)pyrrolo[2,3-b]pyridine with a yield of 60.9%. ¹H NMR (600 MHz, DMSO-d₆) δ 9.27 (s, 2H), 9.07 (s, 1H), 8.57 (d, *J =* 2.5 Hz, 1H), 8.46 (d, *J =* 2.4 Hz, 1H), 8.18 (d, *J =* 2.7 Hz, 1H), 7.69 (d, *J* = 8.2 Hz, 2H), 7.06 (d, *J =* 8.2 Hz, 2H), 3.23 (s, 4H), 2.60 (d, *J =* 20.1 Hz, 4H), 2.32 (s, 3H).¹³C NMR (151 MHz, DMSO) δ 155.32, 153.60, 148.06, 142.01, 129.18, 127.63, 125.96, 124.54, 116.82, 115.64, 107.50, 97.43, 54.13, 47.61, 41.17, 31.17, 28.90. ESI-MS *m*/*z* 525.4 [M+H]⁺.

### 2) Synthesis of compound 5-[4-(4-methylpiperazin-1-yl)phenyl]-3-(pyrimidin-5-yl)-1H-pyrrolo[2,3-b]pyridine

Into a 10 mL single-necked flask, 1-[(4-methylphenyl)dioxo-16-thio]-5-[4-(4-methylpiperazin-1-yl)phenyl]-3-(pyrimidin-5-yl)pyrrolo[2,3-b]pyridine (1.0 eq., 0.16 mmol, 84 mg) and potassium carbonate (3.0 eq., 0.47 mmol, 66 mg) were added, and 5 mL of methanol was added. The mixture was allowed to react at 80°C for 4 h. After concentration under reduced pressure, it was purified by column chromatography (DCM:MeOH=10:1) to obtain 39 mg of the compound 5-[4-(4-methylpiperazin-1-yl)phenyl]-3-(pyrimidin-5-yl)-1*H*-pyrrolo[2,3-*b*]pyridine with a yield of 67.3%. ¹H NMR (600 MHz, DMSO-d₆) δ 9.27 (s, 2H), 9.07 (s, 1H), 8.57 (s, 1H), 8.46 (s, 1H), 8.18 (s, 1H), 7.69 (d, *J =* 8.2 Hz, 2H), 7.05 (d, *J=* 8.2 Hz, 2H), 3.23 (s, 4H), 2.58 (s, 4H), 2.30 (d, *J =* 13.0 Hz, 3H).¹³C NMR (151 MHz, DMSO) δ 155.33, 153.61, 149.91, 148.08, 142.03, 129.22, 128.98, 127.90, 127.64, 125.97, 125.38, 124.55, 116.83, 115.64, 107.51, 54.19, 47.60, 45.24. ESI-MS *m*/*z* 371.3 [M+H]⁺. ESI-HRMS (m/z): Calculated for [C₂₂H₂₂N₆]⁺: 371.2904, found 371.2906.

### Example 24: Synthesis of compound 24, 5-[4-(4-methylpiperazin-1-yl)phenyl]-3-(pyridin-4-yl)-1H-pyrrolo[2,3-b]pyridine

### 1) Synthesis of compound 1-[(4-methylphenyl)dioxo-λ⁶-thio]-5-[4-(4-methylpiperazin-1-yl)phenyl]-3-(pyridin-4-yl)pyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), 4-pyridinboronic acid pinacol ester (1.5 eq., 0.39 mmol, 81 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to obtain 85 mg of compound 1-[(4-methylphenyl)dioxo-λ⁶-thio]-5-[4-(4-methylpiperazin-l-yl)phenyl]-3-(pyridin-4-yl)pyrrolo[2,3-*b*]pyridine with a yield of 62.6%. ¹H NMR (600 MHz, Chloroform-*d*) δ 8.70 (s, 1H), 8.70 - 8.67 (m, 2H), 8.20 (d, *J* = 2.4 Hz, 1H), 8.16 (dd, *J =* 8.4, 2.1 Hz, 2H), 8.05 (d, *J =* 2.1 Hz, 1H), 7.54 (q, *J =* 2.2 Hz, 2H), 7.52 - 7.46 (m, 2H), 7.32 (d, *J =* 8.0 Hz, 2H), 7.02 (dd, *J* = 8.6, 2.2 Hz, 2H), 3.33 (s, 4H), 2.69 (s, 4H), 2.43 (s, 3H), 2.39 (d, *J* = 2.2 Hz, 3H).¹³C NMR (151 MHz, CDCl₃) δ 150.78, 150.58, 146.47, 145.62, 144.60, 140.66, 135.07, 133.10, 129.81, 128.31, 128.18, 126.15, 124.67, 121.72, 120.69, 117.47, 116.37, 54.82, 48.43, 45.86, 21.70. ESI-MS *m*/*z* 540.4 [M+H]⁺.

### 2) Synthesis of compound 5-[4-(4-methylpiperazin-1-yl)phenyl]-3-(pyridin-4-yl)-1H-pyrrolo[2,3-b]pyridine

Into a 10 mL single-necked flask, 1-[(4-methylphenyl)dioxo-λ⁶-thio]-5-[4-(4-methylpiperazin-1-yl)phenyl]-3-(pyridin-4-yl)pyrrolo[2,3-*b*]pyridine (1.0 eq., 0.16 mmol, 85 mg) and potassium carbonate (3.0 eq., 0.48 mmol, 67 mg) were added, and 5 mL of methanol was added. The mixture was allowed to react at 80°C for 4 h. After concentration under reduced pressure, it was purified by column chromatography (DCM:MeOH=10:1) to obtain 39 mg of 5-[4-(4-methylpiperazin-1-yl)phenyl]-3-(pyridin-4-yl)-1*H*-pyrrolo[2,3-*b*]pyridine with a yield of 66.1%. ¹H NMR (600 MHz, DMSO-d₆) δ 8.56 (d, *J =* 5.4 Hz, 3H), 8.48 (s, 1H), 8.22 (s, 1H), 7.83 (d, *J =* 5.2 Hz, 2H), 7.66 (d, *J* = 8.3 Hz, 2H), 7.07 (d, *J =* 8.4 Hz, 2H), 3.22 (s, 4H), 2.53 (s, 4H), 2.29 (s, 3H). ¹³C NMR (151 MHz, DMSO) δ 149.83, 148.23, 142.25, 141.85, 129.26, 128.91, 127.56, 126.78, 124.60, 120.23, 116.88, 115.62, 111.41, 54.22, 47.65, 45.36. ESI-MS *m*/*z* 370.3 [M+H]⁺. ESI-HRMS (m/z): Calculated for [C₂₃H₂₃N₅]⁺: 370.3231, found 370.3234.

### Example 25: Synthesis of compound 25, 5-[4-(4-methylpiperazin-1-yl)phenyl]-3-phenyl-1H-pyrrolo[2,3-b]pyridine

### 1) Synthesis of compound 1-[(4-methylphenyl)dioxo-λ⁶-thio]-5-[4-(4-methylpiperazin-1-yl)phenyl]-3-phenylpyrrolo[2,3-b]pyridine

Into a 50 mL two-necked flask, 3-iodo-1-[(4-methylphenyl)dioxo-λ⁶-thio]-5-[4-(4-methylpiperazin-1-yl)phenyl]pyrrolo[2,3-*b*]pyridine (1.0 eq., 0.26 mmol, 150 mg), (4,4,5,5-tetramethyl-1,3,2-dioxaboran-2-yl)benzene (1.5 eq., 0.39 mmol, 80 mg), potassium carbonate (3.0 eq., 0.79 mmol, 109 mg), Pd(dppf)Cl₂ (0.05 eq., 0.01 mmol, 10 mg) were added, then 4 mL of 1,4-dioxane and 1 mL of water were added, the atmosphere was replaced with argon three times, and the mixture was heated under reflux to react overnight. After the reaction was cooled, it was purified by column chromatography (DCM:MeOH=20:1) to obtain 88 mg of the compound 1-[(4-methylphenyl)dioxo-λ⁶-thio]-5-[4-(4-methylpiperazin-l-yl)phenyl]-3-phenylpyrrolo[2,3-b]pyridine with a yield of 64.7%. ¹H NMR (600 MHz, DMSO-*d*₆) δ 8.67 (d, *J =* 2.4 Hz, 1H), 8.32 (d, *J =* 2.5 Hz, 1H), 8.21 (d, *J =* 2.2 Hz, 1H), 8.07 (dd, *J =* 8.5, 2.0 Hz, 2H), 7.84 (d, *J =* 7.6 Hz, 2H), 7.64 (d, *J* = 8.4 Hz, 2H), 7.51 (t, *J =* 7.8 Hz, 2H), 7.44 (d, *J =* 8.0 Hz, 2H), 7.40 (t, *J =* 7.7 Hz, 1H), 7.06 (d, *J =* 8.4 Hz, 2H), 3.31 (s, 4H), 2.70 (s, 3H), 2.35 (d, *J =* 2.1 Hz, 4H).¹³C NMR (151 MHz, DMSO) δ 145.80, 145.56, 143.23, 134.47, 132.15, 131.94, 129.96, 129.00, 127.84, 127.54, 127.33, 125.93, 123.74, 120.62, 119.62, 115.68, 53.74, 47.02, 28.71, 21.00. ESI-MS *m*/*z* 523.4 [M+H]⁺.

### 2) Synthesis of compound 5-[4-(4-methylpiperazin-1-yl)phenyl]-3-phenyl-1H-pyrrolo[2,3-b]pyridine

Into a 10 mL single-necked flask, 1-[(4-methylphenyl)dioxo-λ⁶-thio]-5-[4-(4-methylpiperazin-1-yl)phenyl]-3-phenylpyrrolo[2,3-*b*]pyridine (1.0 eq., 0.17 mmol, 88 mg) and potassium carbonate (3.0 eq., 0.51 mmol, 70 mg) were added, and 5 mL of methanol was added. The mixture was allowed to react at 80°C for 4 h. After concentration under reduced pressure, it was purified by column chromatography (DCM:MeOH=10:1) to obtain 40 mg of 5-[4-(4-methylpiperazin-1-yl)phenyl]-3-phenyl-1*H*-pyrrolo[2,3-*b*]pyridine with a yield of 65.4%. ¹H NMR (600 MHz, DMSO-*d*₆) δ 8.51 (d, *J* = 2.1 Hz, 1H), 8.34 (d, *J =* 2.1 Hz, 1H), 7.88 (d, *J =* 2.6 Hz, 1H), 7.80 - 7.75 (m, 2H), 7.64 - 7.59 (m, 2H), 7.45 (t, *J =* 7.6 Hz, 2H), 7.26 (t, *J =* 7.4 Hz, 1H), 7.09 - 7.02 (m, 2H), 3.21 (t, *J* = 4.8 Hz, 4H), 2.55 (s, 4H), 2.29 (s, 3H).¹³C NMR (151 MHz, DMSO) δ 149.87, 148.13, 141.48, 134.96, 129.29, 128.80, 128.72, 127.48, 126.26, 125.53, 124.33, 124.28, 117.19, 115.73, 114.35, 54.26, 47.71, 45.38. ESI-MS *m*/*z* 369.3 [M+H]⁺. ESI-HRMS (m/z): Calculated for [C₂₄H₂₄N₄]⁺: 369.3411, found 369.3410.

### Example 26: Experiment on 7-azaindole compounds alleviating the atrophy of mouse myoblasts (C2C12) induced by mouse colon cancer cells (C26)

C2C12 cells were inoculated in 24-well plates. The culture system was high-glucose DMEM medium containing 10% FBS with 1% penicillin-streptomycin. The plates were placed in a 5% CO₂, 37°C environment. When the cell growth density reached 50%-60%, the culture system was replaced with high-glucose DMEM culture medium containing 2% HS with 1% penicillin-streptomycin. Fresh 2% HS differentiation medium was replaced every 48 hours, and the cells were differentiated into mature cells on the 5th or 6th day. Additionally, C26 cells were inoculated in T75 flasks. The culture system was high-glucose DMEM medium containing 10% FBS with 1% penicillin-streptomycin. The flasks were placed in a 5% CO2, 37°C environment. When subculturing, the cells were subcultured at 6 million cells/flask. After culturing in 20 ml of culture medium for 48 hours, the supernatant was collected and centrifuged at 1000 rpm for 3 mins. The supernatant was collected and centrifuged at 4000 rpm for 10 mins to obtain the C26 supernatant. The C26 supernatant and 2% HS differentiation solution were mixed at a ratio of 1:1 (volume ratio) as the muscle atrophy induction solution. Except for the control group in which 2% HS differentiation solution was added, for the other groups, an equivalent amount of muscular atrophy induction solution was added. One group was used as the model group, and the other groups were used as the treatment and experimental groups.

**Table 1 Samples administered for the myoblast (C2C12) atrophy experiment in Example 26**

| Serial number | Numbering | Experimental sample | Composition |
|---|---|---|---|
| 1 | CT | Control group | 2% HS differentiation solution |
| 2 | C26 medium | Model group | 2% HS differentiation solution + C26 supernatant |
| 3 | C26+1 (2.5µM) | Experimental group 1 | 2% HS differentiation solution + C26 supernatant + 2.5 µM compound 1 |
| 4 | C26+2 (1.25µM) | Experimental group 2 | 2% HS differentiation solution + C26 supernatant + 1.25 µM compound 2 |
| 5 | C26+2 (2.5µM) | Experimental group 3 | 2% HS differentiation solution + C26 supernatant + 2.5 µM compound 2 |
| 6 | C26+2 (5µM) | Experimental group 4 | 2% HS differentiation solution + C26 supernatant + 5 µM compound 2 |
| 7 | C26+3 (2.5µM) | Experimental group 5 | 2% HS differentiation solution + C26 supernatant + 2.5 µM compound 3 |
| 8 | C26+4 (5µM) | Experimental group 6 | 2% HS differentiation solution + C26 supernatant + 5 µM compound 4 |
| 9 | C26+5 (5µM) | Experimental group 7 | 2% HS differentiation solution + C26 supernatant + 5 µM compound 5 |
| 10 | C26+6 (2.5µM) | Experimental group 8 | 2% HS differentiation solution + C26 supernatant + 2.5 µM compound 6 |
| 11 | C26+6 (5µM) | Experimental group 9 | 2% HS differentiation solution + C26 supernatant + 5 µM compound 6 |
| 12 | C26+6 (10µM) | Experimental group 10 | 2% HS differentiation solution + C26 supernatant + 10 µM compound 6 |
| 13 | C26+7 (10µM) | Experimental group 11 | 2% HS differentiation solution + C26 supernatant + 10 µM compound 7 |
| 14 | C26+8 (5µM) | Experimental group 12 | 2% HS differentiation solution + C26 supernatant + 5 µM compound 8 |
| 15 | C26+9 (2.5µM) | Experimental group 13 | 2% HS differentiation solution + C26 supernatant + 2.5 µM compound 9 |
| 16 | C26+10 (1.25µM) | Experimental group 14 | 2% HS differentiation solution + C26 supernatant + 1.25 µM compound 10 |
| 17 | C26+10 (2.5µM) | Experimental group 15 | 2% HS differentiation solution + C26 supernatant + 2.5 µM compound 10 |
| 18 | C26+10 (5µM) | Experimental group 16 | 2% HS differentiation solution + C26 supernatant + 5 µM compound 10 |
| 19 | C26+11 (1.25µM) | Experimental group 17 | 2% HS differentiation solution + C26 supernatant + 1.25 µM compound 11 |
| 20 | C26+11 (2.5µM) | Experimental group 18 | 2% HS differentiation solution + C26 supernatant + 2.5 µM compound 11 |
| 21 | C26+11 (5µM) | Experimental group 19 | 2% HS differentiation solution + C26 supernatant + 5 µM compound 11 |
| 22 | C26+12 (5µM) | Experimental group 20 | 2% HS differentiation solution + C26 supernatant + 5 µM compound 12 |
| 23 | C26+13 (10µM) | Experimental group 21 | 2% HS differentiation solution + C26 supernatant + 10 µM compound 13 |
| 24 | C26+14 (2.5µM) | Experimental group 22 | 2% HS differentiation solution + C26 supernatant + 2.5 µM compound 14 |
| 25 | C26+14 (5µM) | Experimental group 23 | 2% HS differentiation solution + C26 supernatant + 5 µM compound 14 |
| 26 | C26+14 (10µM) | Experimental group 24 | 2% HS differentiation solution + C26 supernatant + 10 µM compound 14 |
| 27 | C26+15 (10µM) | Experimental group 25 | 2% HS differentiation solution + C26 supernatant + 10 µM compound 15 |
| 28 | C26+16 (5µM) | Experimental group 26 | 2% HS differentiation solution + C26 supernatant + 5 µM compound 16 |
| 29 | C26+17 (1.25µM) | Experimental group 27 | 2% HS differentiation solution + C26 supernatant + 1.25 µM compound 17 |
| 30 | C26+17 (2.5µM) | Experimental group 28 | 2% HS differentiation solution + C26 supernatant + 2.5 µM compound 17 |
| 31 | C26+17 (5µM) | Experimental group 29 | 2% HS differentiation solution + C26 supernatant + 5 µM compound 17 |
| 32 | C26+18 (10µM) | Experimental group 30 | 2% HS differentiation solution + C26 supernatant + 10 µM compound 18 |

The diameter of myotubes after differentiation of mouse myoblasts (C2C12) was evaluated by using the diameter measurement method. The method for measuring the diameter of myotubes was as follows: after 48 hours of treatment, the myotubes were fixed with a fixative (anhydrous ethanol : formaldehyde : glacial acetic acid (volume ratio) = 20:2:1) for more than 1 h and stained. The staining method used in the experiment is hematoxylin-eosin staining, referred to as HE staining for short. Hematoxylin stain is alkaline and positively charged, and it can easily bind to the negatively charged and acidic deoxyribonucleic acid (DNA) in the cell nucleus through ionic bonds to stain it blue; eosin is an acidic dye that dissociates into negatively charged anions in water, and it can easily bind to the positively charged amino groups of proteins in the cytoplasm to stain it red. The stained cells were placed under a high-power microscope to take pictures (magnification: 200 times), and the diameter of myotube cells was statistically analyzed using image J software. The muscle atrophy reversal rate was calculated according to the following formula: Muscle atrophy reversal rate = (average value of myotubes in the treatment group - average value of myotubes in the model group) / (average value of myotubes in the control group - average value of myotubes in the model group)× 100%

### Results and discussions:

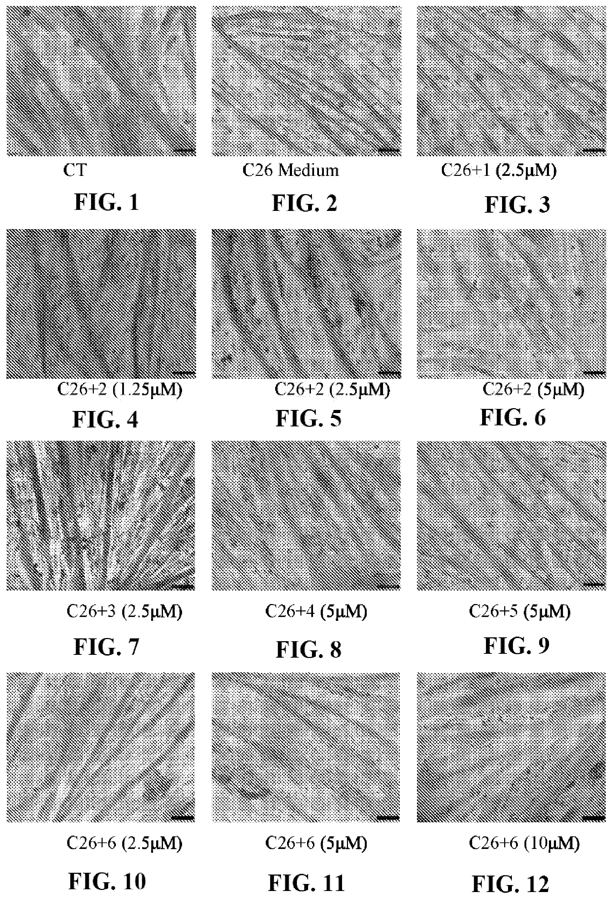

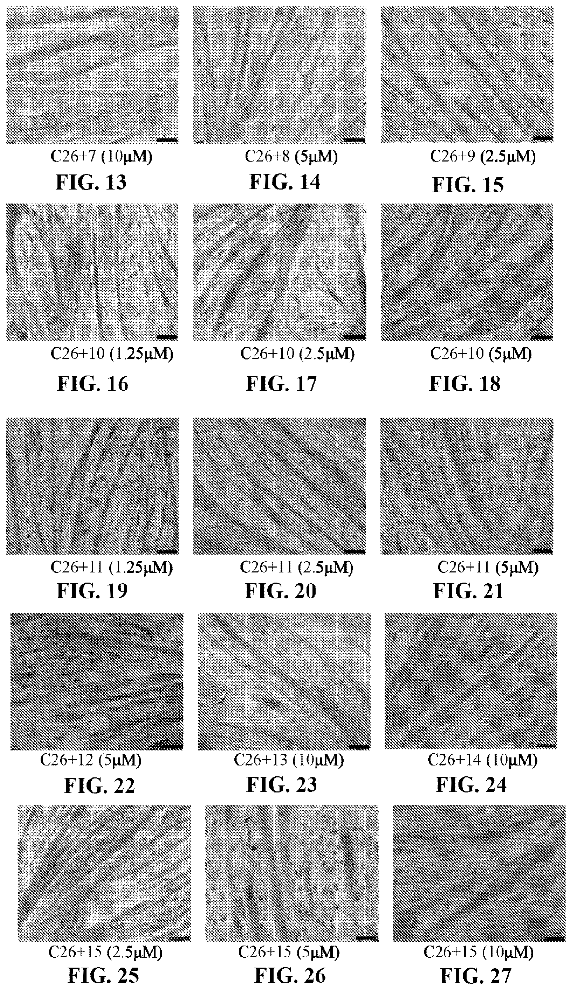

FIGS. 1-32 are representative HE-staining images showing that 7-azaindole compounds alleviate C2C12 mature myotube atrophy induced by C26 cell culture medium, and Table 1 shows myotube statistical results. As shown in FIGS. 1-32 and Table 2, 7-azaindole compounds significantly reverse muscle cell atrophy in a concentration-dependent manner.

**Table 2 Myotube statistical results**

| Compound | Myotube atrophy reversal rate (%) | | | | |
|---|---|---|---|---|---|
| | 0.625µM | 1.25µM | 2.5µM | 5µM | 10µM |
| **1** | - | - | 63.55 | - | - |
| **2** | - | 69.20 | 97.61 | 130.63 | |
| **3** | - | - | 90.87 | - | - |
| **4** | - | - | - | 63.64 | - |
| **5** | - | - | - | 79.48 | - |
| **6** | - | - | 45.91 | 79.11 | 99.78 |
| **7** | - | - | - | - | 77.09 |
| **8** | - | - | - | 62.19 | - |
| **9** | - | - | 61.15 | - | - |
| **10** | - | 42.43 | 58.85 | 100.3 | - |
| **11** | - | 49.58 | 55.29 | 102.59 | - |
| **12** | - | - | - | 73.77 | - |
| **13** | - | - | - | - | 86.17 |
| **14** | - | - | - | - | 57.04 |
| **15** | - | - | 47.52 | 99.06 | 109.24 |
| **16** | - | - | - | 78.02 | - |
| **17** | - | 39.92 | 64.99 | 97.66 | - |
| **18** | - | - | - | - | 84.54 |
| **19** | - | - | - | 69.36 | - |
| **20** | - | 54.04 | - | - | - |
| **21** | 44.75 | - | - | - | - |
| **22** | - | - | - | 75.38 | - |
| **23** | - | - | - | 97.91 | - |
| **24** | - | - | - | 76.74 | - |
| **25** | - | - | - | 77.08 | - |

### Example 27:

### Test of the inhibitory activity of 7-azaindole compounds on ActRIIB

The ATP and substrate concentrations recommended by the ADP-Glo^{™} assay kit were used, which were 25 µM and 0.1 mg/mL, respectively, and a series of isocratic final ActRIIB concentrations were set up, which were 0, 0.47, 0.94, 1.88, 3.75, 7.5, 15, 30, and 60 ng/µL, respectively. According to the fluorescence values obtained in the experiment, an ActRIIB concentration titration curve was drawn. The ActRIIB concentrations corresponding to the ratio of the signal value to the background value (i.e., signal-to-background ratio, SBR) in the range of 5-10 times are suitable for screening compounds for inhibition of ActRIIB activity. From the ActRIIB concentration titration curve, the final ActRIIB concentrations corresponding to SB5 and SB10 were calculated to be 14.9 and 23.7 ng/µL, respectively. Finally, a final concentration of 20.0 ng/µL was selected as the experimental concentration for screening compounds for their inhibitory activity against ActRIIB. The inhibitory efficiency of 7-azaindole compounds on ActRIIB was detected at concentrations of 0.1, 1, 10, and 100 nM, respectively. The inhibition curves were drawn and the IC₅₀ values were calculated, as shown in Table 3.

**Table 3 IC₅₀ values of 7-azaindole compounds against ActRIIB**

| Compound | ActRIIB IC₅₀ (nM) |
|---|---|
| **1** | 20.4 |
| **2** | 1.8 |
| **3** | 1.1 |
| **4** | 58.61 |
| **5** | 1.2 |
| **6** | 1.4 |
| **7** | 4.4 |
| **8** | 2.6 |
| **9** | 70.8 |
| **10** | 4.0 |
| **11** | 2.2 |
| **13** | 12.64 |
| **16** | 1.86 |
| **17** | 3.61 |
| **18** | 78.86 |
| **19** | 24.2 |
| **21** | 103.0 |
| **22** | 87.3 |
| **23** | 4.8 |
| **24** | 59.3 |
| **25** | 1.2 |

### Example 28:

### Experimental results of compound in treating cancer cachexia animal model

1. For the experimental results of 7-azaindole compound 2 in treating the cancer cachexia animal model, the method was as follows:
   C26 cells were inoculated in T75 flasks. The culture system was 1640 medium containing 10% FBS with 1% penicillin-streptomycin. The flasks were placed in a 5% CO₂, 37°C environment for expansion culture. The cells were collected by centrifugation at 1000 rpm for 3 mins, and the culture medium was washed away with ice-cold PBS buffer, and a cell suspension of 10 million/ml was prepared. The cell suspension was inoculated into the left and right armpits of BALB/c mice at a concentration of 1 million for tumor development. When the tumor volume increased to 1000 mm³, the tumor was removed and homogenized with 3 ml of ice saline/g to obtain a tumor tissue suspension. The mice to be inoculated were divided into groups according to their weight, and the cell suspension was inoculated into the left armpit of Balb/c mice at an inoculation volume of 100 µl per mouse. Treatment was started from the day after inoculation. Compound 2 was dissolved in 3% DMSO, 2% Solutol and 95% saline in sequence to form a uniform solution with the required concentration. The solution was prepared and used daily. The dose was 5 mg/kg and the compound was administered by the intragastric (ig) route. The body weight, temperature, tumor size and food intake of mice were monitored daily. In the early stage of the experiment, the tumor volume of mice in the model group was small and their body weight did not decrease, which was the pre-cachexia stage. When the tumor volume of mice in the model group reached 2.0 × 10³ mm³ or their body weight decreased by 10%, they were considered to have entered the cachexia stage. The muscle grasp force of the mice's forelimbs was tested at these two stages. The grasp force of the mice's limb muscles was tested at these two stages. At the end of the experiment, the mice were killed by cervical dislocation and the gastrocnemius, tibialis anterior, right hind limb and tumor samples were obtained, and the sample weights were measured.

The method for detecting muscle grasp force was as follows: The muscle grasp force of mice was tested on the 5th day (pre-cachexia stage) and the 22nd day (cachexia stage) of the experiment. The mouse was steadily placed on the YLS-13A Mouse and Rat Grasp Force Tester by using the right hand, so that its forelimbs could tightly grasp the gripping disk, and the gripping disk was stabilized by using the left hand moving forward. When the left hand slowly released the gripping disk, the right hand immediately and slowly pulled the mouse's tail backwards until its forelimbs were separated from the gripping disk. The test was repeated 8 times for each mouse, and the mean value of the 8 measurements was used as the skeletal muscle strength index of each mouse.

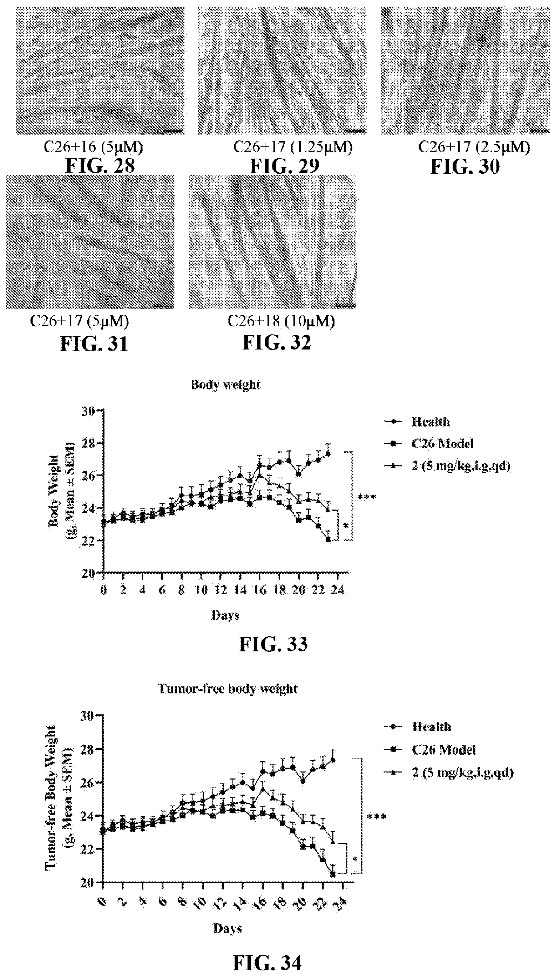

Results and conclusions: Refer to FIGS. 33-45. The three curves in the figures represent: healthy group, C26 tumor model group and treatment group (compound 2 group, in which the dose is 5 mg/kg), respectively.

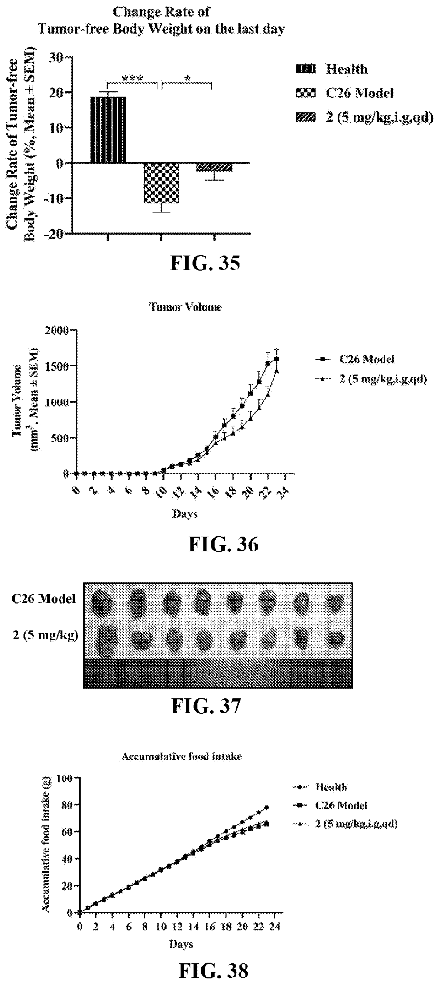

FIGS. 31-35 show the tumor-bearing body weight of the mice during their survival period (FIG. 33), the tumor-free body weight (FIG. 34), the tumor-free body weight change rate (FIG. 35), tumor volume (FIG. 36) and tumor photographs (FIG. 37) on the last day of the mice, wherein the tumor-bearing body weight and tumor-free body weight are the average body weights of 8 mice, the tumor-free body weight change rate is the average body weight change rate of 8 mice, the tumor volume is the average volume of the tumors of 8 mice, and the tumor photographs are tumor photographs of 8 mice. As shown in FIG. 33, the body weight of mice in the healthy group continued to increase; while the body weight of mice in the C26 tumor model group, from the beginning of the experiment, began to decrease on the 15th day, and continued to decrease until the end of the experiment, as shown in FIG. 34, the same was true for the tumor-free body weight; and the compound 2 group was able to significantly alleviate the weight loss of mice, and by the end of the experiment, both the tumor-bearing body weight and the tumor-free body weight were higher than those of the C26 tumor model group, with statistically significant differences (p<0.05). However, as shown in FIGS. 36 and 37, the tumor volume of the compound 2 group was slightly reduced compared with the C26 tumor model group, but there was no significant difference, indicating that compound 2 has no obvious inhibitory effect on C26 tumors.

FIG. 38 shows the average accumulative food intake of mice during their survival period, which is the average calculation result of 8 mice. The food intake of mice in the C26 tumor model group was significantly lower than that in the healthy group. The food intake of the compound 2 group was slightly higher than that in the C26 tumor model group, the appetite was slightly improved.

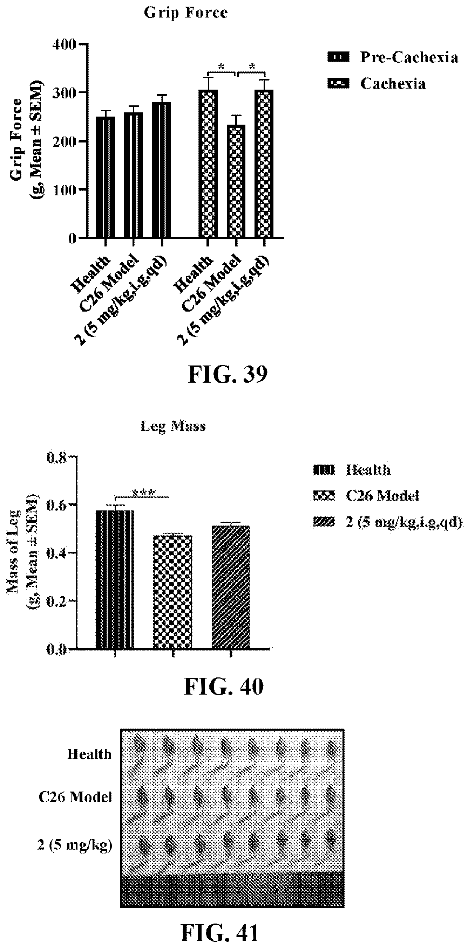

FIG. 39 shows the muscle grasp force of the mouse limbs. The muscle grasp force of the mice in the cachexia C26 tumor model group was significantly lower than that in the healthy group (p=0.06). Compound 2 could significantly enhance the muscle grasp force of cachexia mice (p=0.06).

FIGS. 40 to 41 show the weight of the right hind limb of mice and the photographs of the right hind limb. The weight of the right hind limb is the average value of 8 mice, and the photographs of the right hind limb are the results of 8 mice/group. As shown in FIGS. 40 and 41, the weight of the right hind limbs of mice in the C26 tumor model group was significantly lower than that in the healthy group, and the difference was statistically significant (p<0.001). Compound 2 can improve skeletal muscle atrophy in cachectic mice to a certain extent.

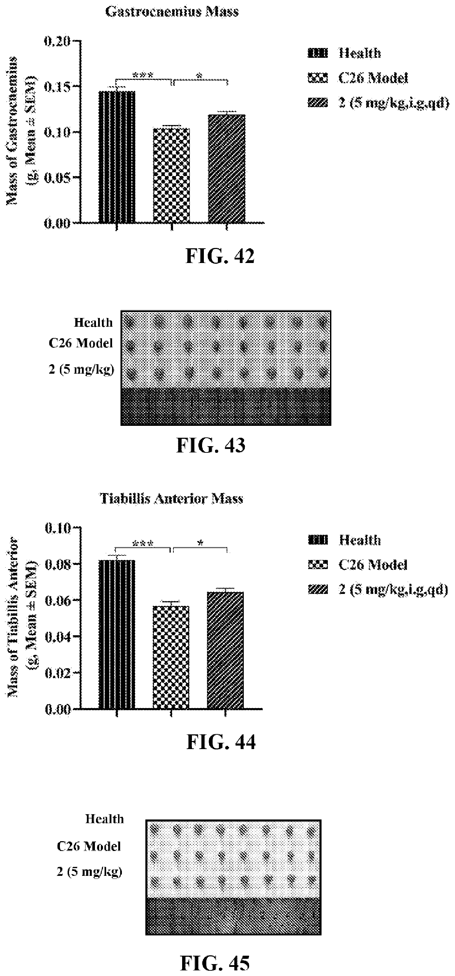

FIGS.s 42 to 45 show the gastrocnemius mass, gastrocnemius photographs, tibialis anterior mass, and tibialis anterior photographs of the mice. The gastrocnemius mass and tibialis anterior mass are the average values of 8 mice, and the photographs of the gastrocnemius and tibialis anterior are the results of 8 mice/group. As shown in FIGS. 42 and 43, the gastrocnemius mass of mice in the C26 tumor model group was significantly smaller than that in the healthy group, and the difference was statistically significant (p<0.001). Compound 2 can significantly alleviate the gastrocnemius atrophy in cachectic mice (p<0.05). As shown in FIGS. 44 and 45, the tibialis anterior mass of mice in the C26 tumor model group was significantly smaller than that in the healthy group, and the difference was statistically significant (p<0.001). Compound 2 can significantly alleviate the tibialis anterior atrophy in cachectic mice (p<0.05).

The above results indicate that compound 2 can alleviate weight loss, muscle atrophy, muscle strength caused by cancer cachexia without affecting tumor size, and slightly improves appetite.

2. For the experimental results of 7-azaindole compounds 3 and 10 in treating the cancer cachexia animal model, the method was as follows:

C26 cells were inoculated in T75 flasks. The culture system was 1640 medium containing 10% FBS with 1% penicillin-streptomycin. The flasks were placed in a 5% CO₂, 37°C environment for expansion culture. The cells were collected by centrifugation at 1000 rpm for 3 mins, and the culture medium was washed away with ice-cold PBS buffer, and a cell suspension of 10 million/ml was prepared. The cell suspension was inoculated into the left and right armpits of BALB/c mice at a concentration of 1 million for tumor development. When the tumor volume increased to 1000 mm³, the tumor was removed and homogenized with 3 ml of ice saline/g to obtain a tumor tissue suspension. The mice to be inoculated were divided into groups according to their weight, and the cell suspension was inoculated into the left armpit of BALB/c mice at an inoculation volume of 100 µl per mouse. Treatment was started from the day after inoculation. Compounds 3 and 10 were dissolved in 3% DMSO, 2% Solutol and 95% saline in sequence to form a uniform solution with the required concentration. The solution was prepared and used daily. The dose was 10 mg/kg and the compound was administered by intraperitoneal injection (ip). The body weight, temperature, tumor size and food intake of mice were monitored daily. In the early stage of the experiment, the tumor volume of the mice in the model group was small and their body weight did not decrease, which was the pre-cachexia stage. In the middle stage of the experiment, the weight of the mice in the model group began to decrease, which was considered to have entered the mid-cachexia stage. When the tumor volume of the mice in the model group reached 2.0 × 10³ mm³ or their body weight decreased by 10%, they were considered to have entered the late-cachexia stage. The muscle grasp force of the mice's forelimbs was tested at these three stages. At the end of the experiment, the mice were killed by cervical dislocation and the gastrocnemius, tibialis anterior, right hind limb and tumor samples were obtained, and the sample weights were measured. Gastrocnemius tissue of appropriate size was obtained, and H&E stained tissue sections were prepared through processes such as fixation, embedding, slicing, staining. At least 10 fields of view were randomly selected for slices of different groups for photographing, and the cross-sectional areas of all muscle fibers in the photographs were measured and statistically analyzed.

The method for detecting muscle grasp force was as follows: The muscle grasp force of mice was tested on the 3rd day (pre-cachexia stage), the 11th day (mid-cachexia stage) and the 16th day (late-cachexia stage) of the experiment. The mouse was steadily placed on the YLS-13A Mouse and Rat Grasp Force Tester by using the right hand, so that its forelimbs could tightly grasp the gripping disk, and the gripping disk was stabilized by using the left hand moving forward. When the left hand slowly released the gripping disk, the right hand immediately and slowly pulled the mouse's tail backwards until its forelimbs were separated from the gripping disk. The test was repeated 8 times for each mouse, and the mean value of the 8 measurements was used as the skeletal muscle strength index of each mouse.

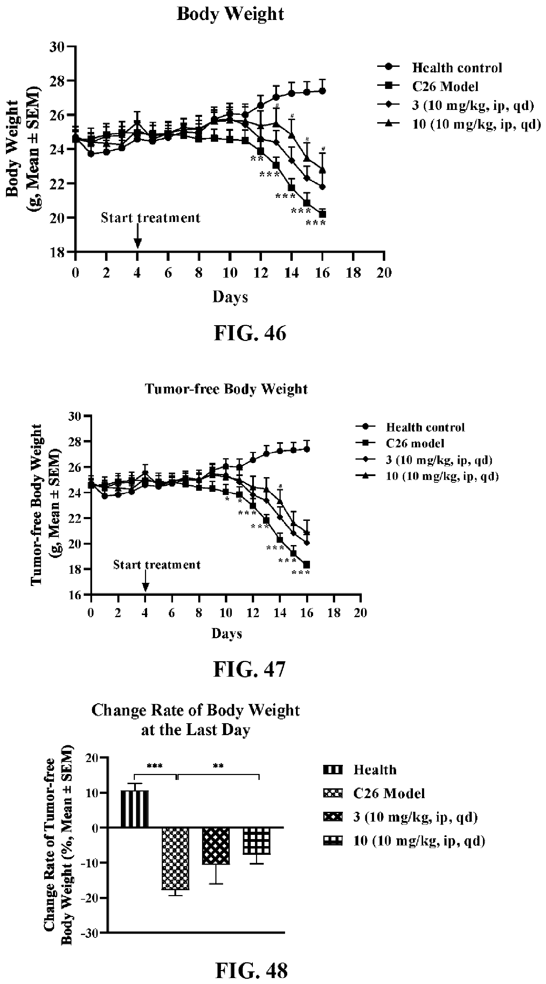

Results and conclusions: Refer to FIGS. 46-58. The four curves in the figure represent: healthy group, C26 tumor model group, compound 3 treatment group (dose, 10 mg/kg) and compound 10 treatment group (dose, 10 mg/kg).

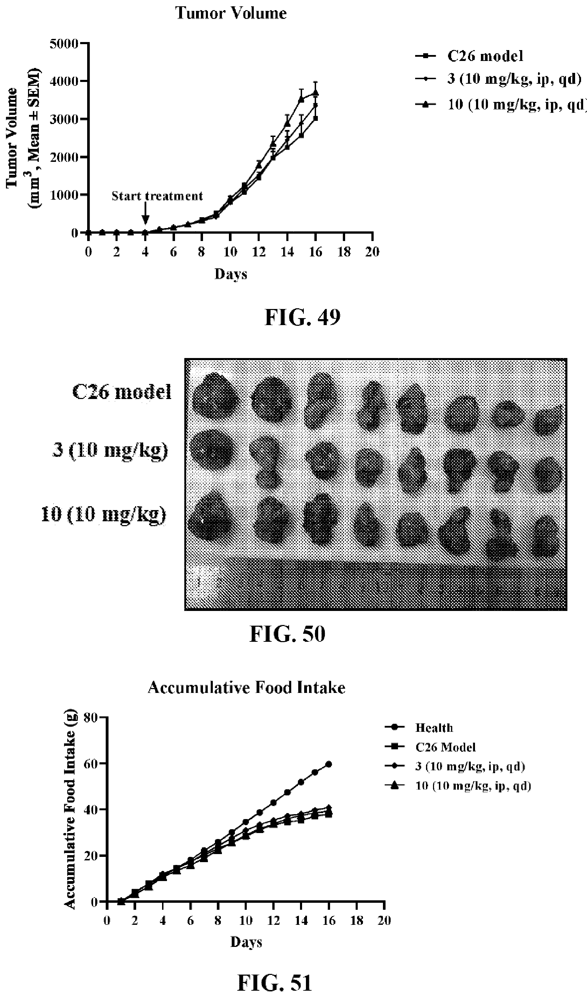

FIGS. 46-48 show the tumor-bearing body weight of the mice during their survival period (FIG. 46), the tumor-free body weight (FIG. 47), the tumor-free body weight change rate (FIG. 48), tumor volume (FIG. 49) and tumor photographs (FIG. 50) on the last day of the mice, wherein the tumor-bearing body weight and tumor-free body weight are the average body weights of 8 mice, the tumor-free body weight change rate is the average body weight change rate of 8 mice, the tumor volume is the average volume of the tumors of 8 mice, and the tumor photographs are tumor photographs of 8 mice. As shown in FIG. 46, the body weight of mice in the healthy group continued to increase; while the body weight of mice in the C26 tumor model group, from the beginning of the experiment, began to decrease on the 11th day, and continued to decrease until the end of the experiment, as shown in FIG. 47, the same was true for the tumor-free body weight; and the compound 3 group was able to alleviate the weight loss of mice to some extent, and by the end of the experiment, both the tumor-bearing body weight and the tumor-free body weight were higher than those of the C26 tumor model group, but the difference was not statistically significant. The compound 10 group was able to significantly alleviate the weight loss of mice. At the end of the experiment, both the tumor-bearing body weight and the tumor-free body weight were higher than those of the C26 tumor model group, and the differences were statistically significant (p<0.05). However, as shown in FIGS. 49 and 50, the tumor volume of the compound 3 group and the compound 10 group was slightly reduced compared with the C26 tumor model group, but there was no significant difference, indicating that compounds 3 and 10 have no obvious inhibitory effect on C26 tumors.

FIG. 51 shows the average accumulative food intake of mice during their survival period, which is the average calculation result of 8 mice. The food intake of mice in the C26 tumor model group was significantly lower than that in the healthy group. The food intake of the compound 3 group and compound 10 group was slightly higher than that in the C26 tumor model group, the appetite was slightly improved.

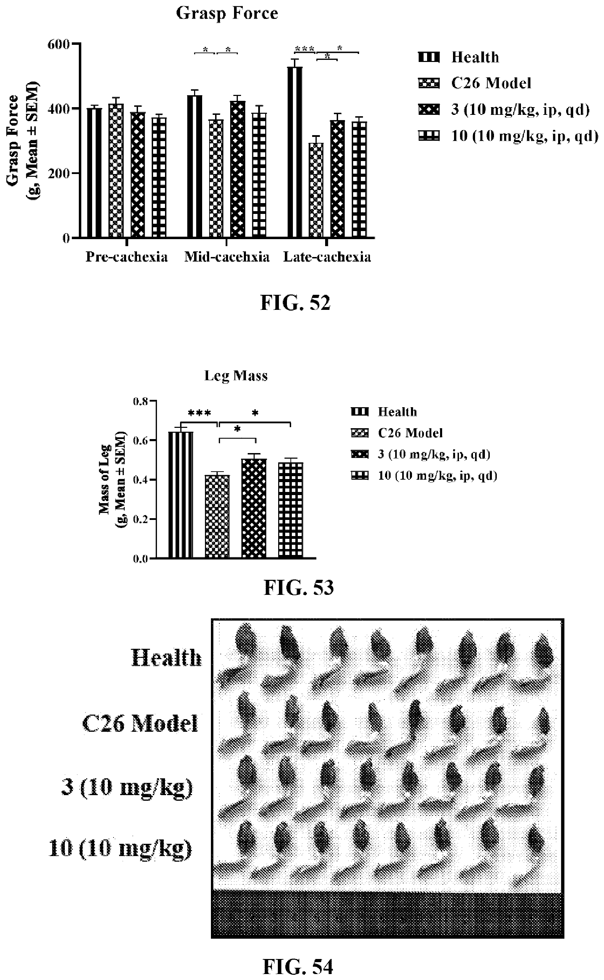

FIG. 52 shows the muscle grasp force of the mouse limbs. The muscle grasp force of the mice in the middle and late cachexia C26 tumor model groups was significantly lower than that in the healthy group (p<0.05). Compounds 3 and 10 could significantly enhance the muscle grasp force of cachexia mice (p<0.05).

FIGS. 53 to 54 show the weight of the right hind limb of mice and the photographs of the right hind limb. The weight of the right hind limb is the average value of 8 mice, and the photographs of the right hind limb are the result of 8 mice/group. As shown in FIGS. 53 and 54, the weight of the right hind limbs of mice in the C26 tumor model group was significantly lower than that in the healthy group, and the difference was statistically significant (p<0.05). Compounds 3 and 10 can improve skeletal muscle atrophy in cachectic mice to a certain extent.

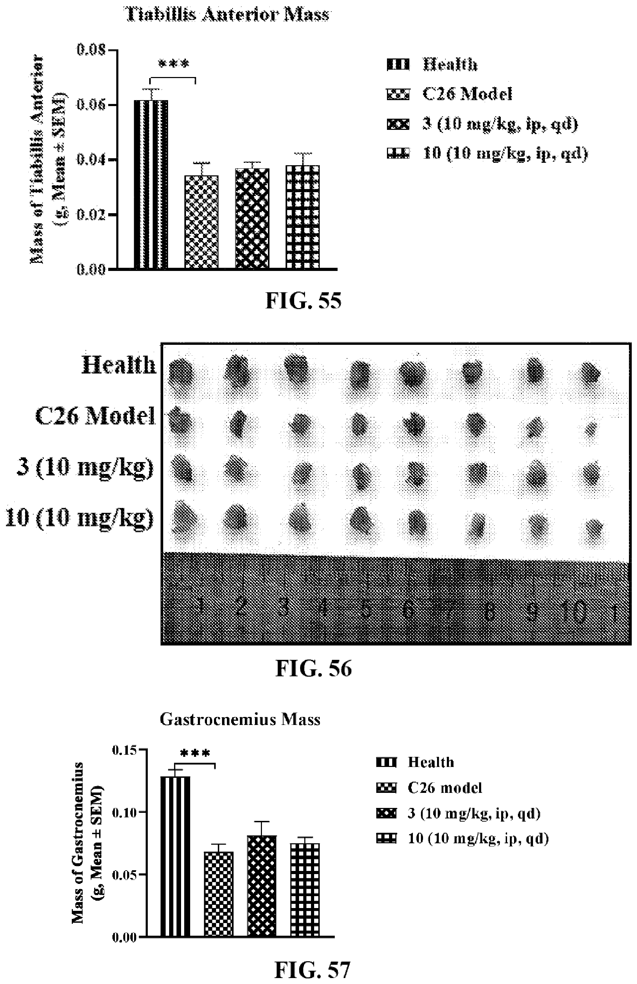

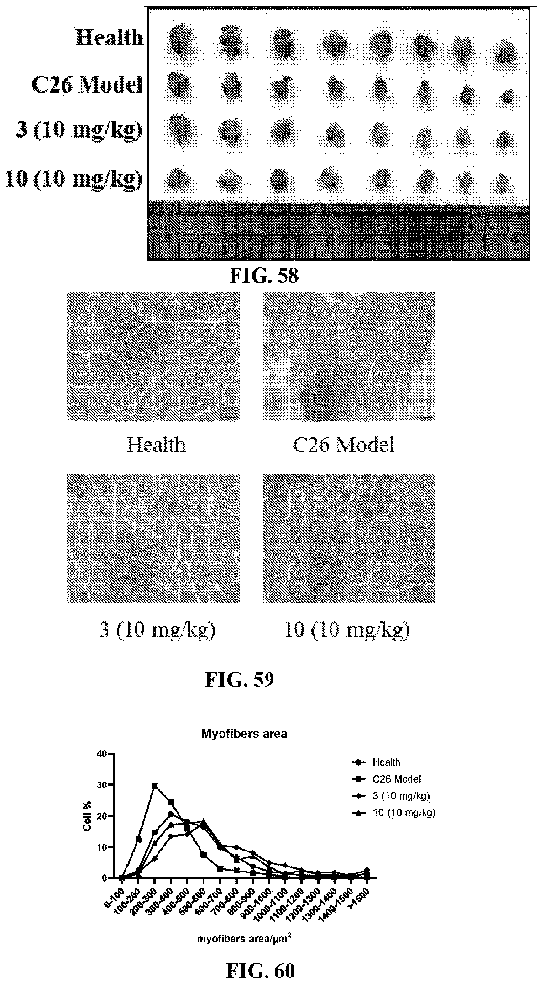

FIGS. 55 to 60 show the mice tibialis anterior mass, tibialis anterior photographs, gastrocnemius mass, gastrocnemius photographs, statistical graphs of the cross-sectional distribution of gastrocnemius fibers, and schematic diagrams of gastrocnemius tissue sections. The tibialis anterior mass and the gastrocnemius mass are the average values of 8 mice, the photographs of the tibialis anterior and gastrocnemius are the results of 8 mice/group, and the statistical graphs of the cross-sectional distribution of gastrocnemius fibers are the statistical results of 3 mice. As shown in FIGS. 55 and 56, the tibialis anterior mass of mice in the C26 tumor model group was significantly smaller than that in the healthy group, and the difference was statistically significant (p<0.001). Compounds 3 and 10 cannot significantly alleviate the gastrocnemius atrophy in cachectic mice. As shown in FIGS. 57 and 58, the gastrocnemius mass of mice in the C26 tumor model group was significantly smaller than that in the healthy group, and the difference was statistically significant (p<0.001). Compounds 3 and 10 cannot significantly alleviate the tibialis anterior atrophy in cachectic mice. As shown in FIGS. 59 and 60, the cross-sectional area of the gastrocnemius fibers of mice in the C26 tumor model group was significantly smaller than that in the healthy group, and compounds 3 and 10 could significantly alleviate the reduction in the cross-sectional area of the gastrocnemius in cachectic mice.

The above results indicate that compounds 3 and 10 can alleviate weight loss, muscle atrophy, muscle strength caused by cancer cachexia without affecting tumor size, and slightly improve appetite.

The above are preferred embodiments of the present invention. It should be pointed out that those of ordinary skill in the technical field covered by the present invention can make several supplements and improvements without departing from the method of the present invention. These supplements and improvements should also be regarded as the scope of protection of the present invention.

## Claims

1. A 7-azaindole compound, having a structural formula represented by formula (I): 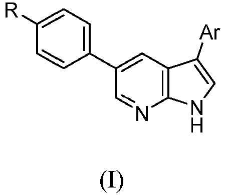 or a pharmaceutically acceptable salt thereof, wherein:
Ar is selected from pyrazole, indole or pyrazole containing substituents; in the pyrazole containing substituents, the substituent is selected from C1-4 hydrocarbon groups, and the number of substituents is 1 or 2;
R is selected from 4-methylpiperazine, piperazine, morpholine,

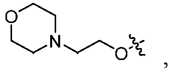

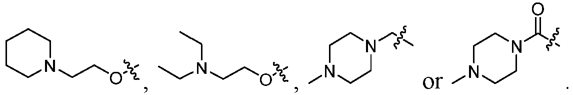

2. A 7-azaindole compound, having a structural formula represented by formula (II): 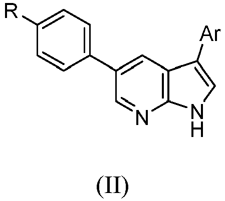 or a pharmaceutically acceptable salt thereof, wherein:
Ar is selected from pyrazole, indole, pyrazole containing substituents, isoxazole containing substituents, pyrimidine or 3-pyridyl; in the pyrazole containing substituents and the isoxazole containing substituents, the substituent is selected from C1-4 hydrocarbon groups, and the number of substituents is 1 or 2;
R is selected from 4-methylpiperazine, piperazine, morpholine, 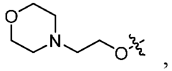 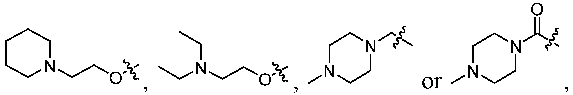
with the restrictions that:
a) when R is morpholine, Ar is not 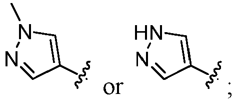
b) when R is 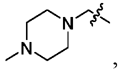 Ar is not 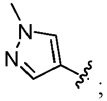
c) when Ar is indole, R is not

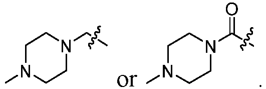

3. A 7-azaindole compound, having a structural formula represented by formula **(III):** 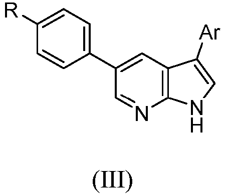 or a pharmaceutically acceptable salt thereof, wherein:
Ar is selected from pyrazole, indole, pyrazole containing substituents, isoxazole containing substituents, pyrimidine or 3-pyridine; in the pyrazole containing substituents and the isoxazole containing substituents, the substituent is selected from C1-4 hydrocarbon groups, and the number of substituents is 1 or 2;
R is selected from 4-methylpiperazine, piperazine, 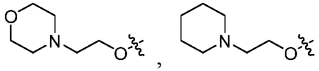
or

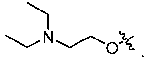

4. The 7-azaindole compound or pharmaceutically acceptable salt thereof according to any one of claims 1-3, wherein the Ar is indole.

5. The 7-azaindole compound or pharmaceutically acceptable salt thereof according to any one of claims 1-3, wherein the Ar is pyrazole or pyrazole containing substituents.

6. The 7-azaindole compound or pharmaceutically acceptable salt thereof according to any one of claims 1-3, wherein the Ar is selected from

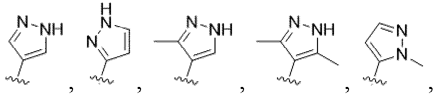

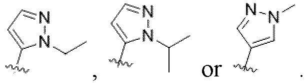

7. The 7-azaindole compound or pharmaceutically acceptable salt thereof according to any one of claims 1-6, wherein the R is 4-methylpiperazine.

8. The 7-azaindole compound or pharmaceutically acceptable salt thereof according to any one of claims 1-7 for use in inhibiting ActRIIB activity.

9. The 7-azaindole compound or pharmaceutically acceptable salt thereof according to any one of claims 1-7 for use in treating cachexia.

10. A pharmaceutical composition, comprising the 7-azaindole compound or pharmaceutically acceptable salt thereof according to any one of claims 1-7, and a pharmaceutically acceptable additive.

11. The pharmaceutical composition according to claim 10, wherein the pharmaceutically acceptable additive is selected from pharmaceutically acceptable carriers, diluents or excipients.

12. Use of the 7-azaindole compound or pharmaceutically acceptable salt thereof according to any one of claims 1-7, and the pharmaceutical composition according to claims 10-11 in the preparation of an ActRIIB inhibitor.

13. Use of the 7-azaindole compound or pharmaceutically acceptable salt thereof according to any one of claims 1-7, and the pharmaceutical composition according to claims 10-11 in the preparation of anti-cachexia drugs, especially anti-cancer-cachexia drugs.

14. A pharmaceutical composition, comprising a compound represented by structural formula **(IV):** 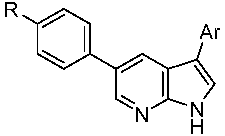 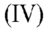 or a pharmaceutically acceptable salt thereof, and a pharmaceutically acceptable additive, preferably the additive being selected from a pharmaceutically acceptable carrier, diluent or excipient; wherein:
Ar is selected from pyrazole, indole, pyrazole containing substituents, isoxazole containing substituents, pyrimidine or 3-pyridine; in the pyrazole containing substituents and the isoxazole containing substituents, the substituent is selected from C1-4 hydrocarbon groups, and the number of substituents is 1 or 2;
R is selected from 4-methylpiperazine, piperazine, morpholine, 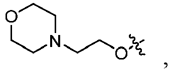 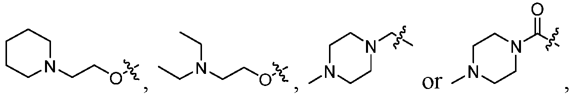
with the restrictions that:
a) when R is morpholine, Ar is not 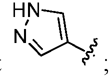
b) when R is 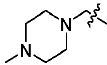 , Ar is not 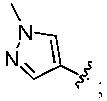
c) when Ar is indole, R is not

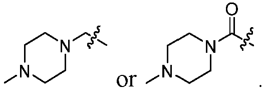

15. The 7-azaindole compound according to any one of claims 1-9, the pharmaceutical composition according to any one of claims 10, 11 and 14, and the use according to any one of claims 12 and 13, wherein the 7-azaindole compound is selected from any one of the following compounds: 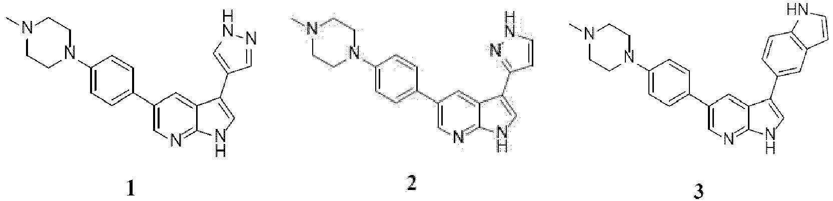 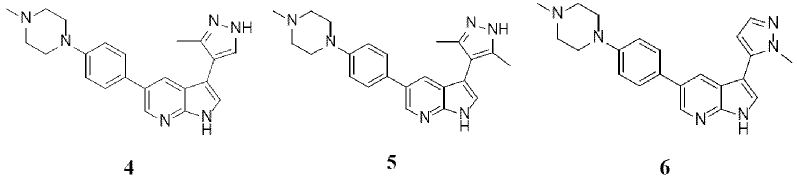 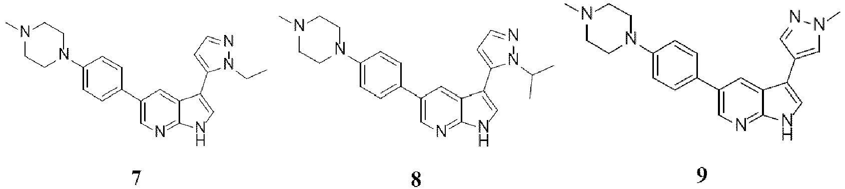 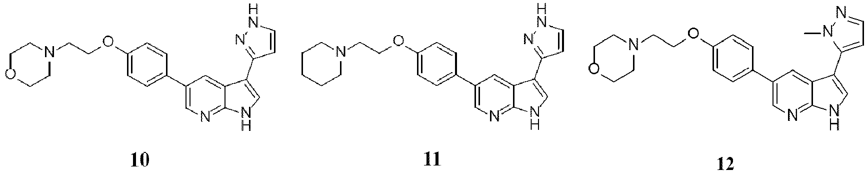 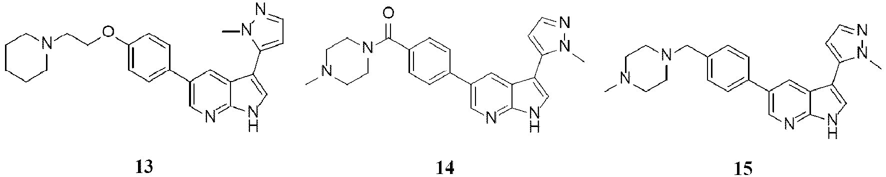 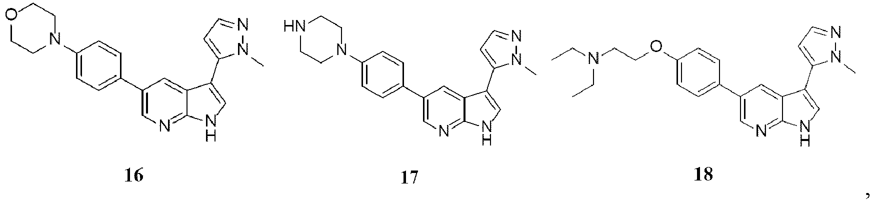 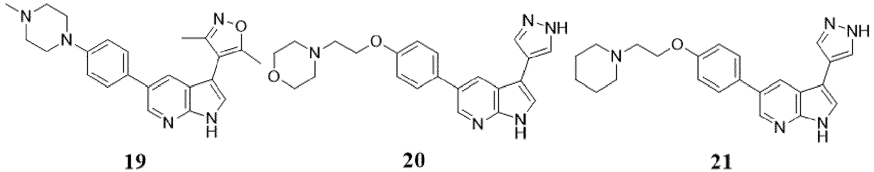 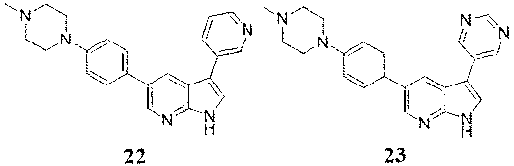 or a pharmaceutically acceptable salt thereof.

16. Use of a compound represented by structural formula **(V):** 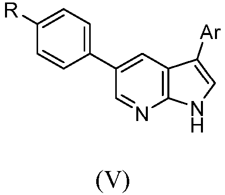 or a pharmaceutically acceptable salt thereof in the preparation of anti-cachexia drugs, wherein:
Ar is selected from pyrazole, indole, pyrazole containing substituents, isoxazole containing substituents, pyrimidine, pyridine or phenyl; in the pyrazole containing substituents and the isoxazole containing substituents, the substituent is selected from C1-4 hydrocarbon groups, and the number of substituents is 1 or 2;
R is selected from 4-methylpiperazine, piperazine, morpholine, 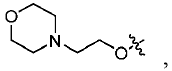 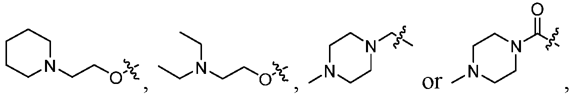
with the restrictions that:
a) when R is morpholine, Ar is not 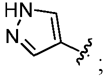
b) when R is 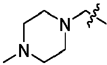 , Ar is not 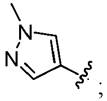
c) when Ar is indole, R is not

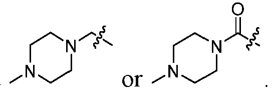

17. The use of the compound according to claim 16, wherein the compound is selected from any one of the following compounds: 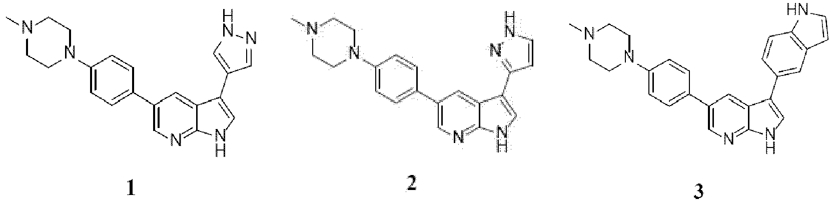 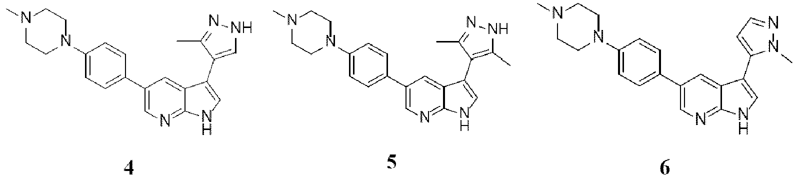 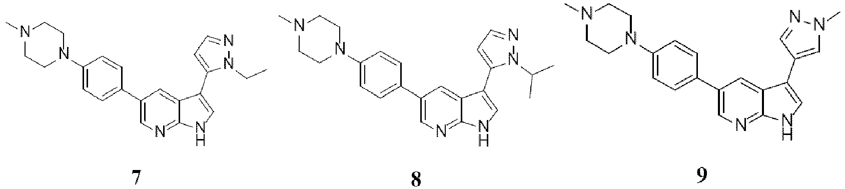 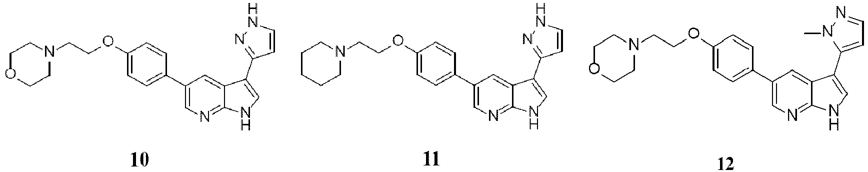 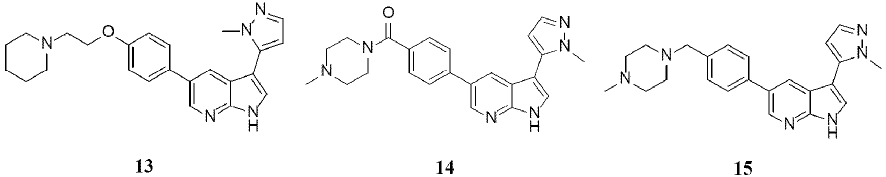 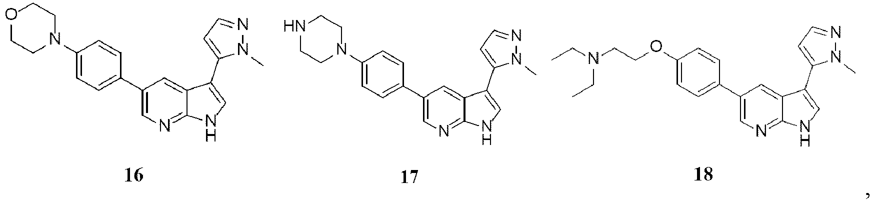 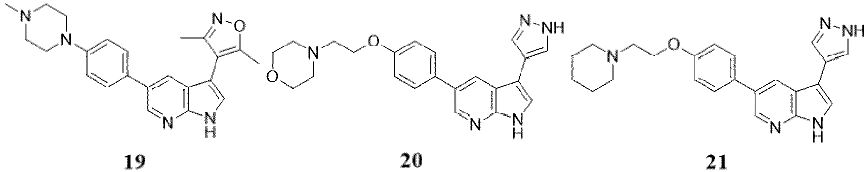 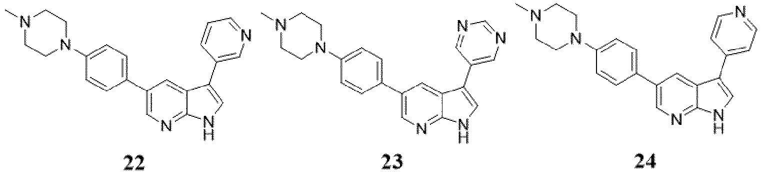 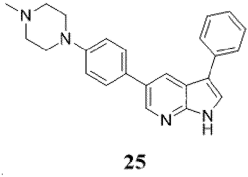 or a pharmaceutically acceptable salt thereof.
